# EUROPEAN PATENT SPECIFICATION

(11) **EP 2 959 020 B1**
(45) Date of publication and mention of the grant of the patent: **18.09.2019**
(21) Application number: 14754622.0
(22) Date of filing: 20.02.2014
(51) Int. Cl.: C12Q 1/6809, C12Q 1/6858, C12Q 1/6869

(54) **METHOD TO SELECT RARE CLONOTYPES**
VERFAHREN ZUR AUSWAHL VON SELTENE KLONOTYPEN
PROCÉDÉ POUR SÉLECTIONNER DES CLONOTYPES RARES

(30) Priority: 22.02.2013 US 201361768269 P; 15.03.2013 US 201313834794
(43) Date of publication of application: 30.12.2015
(73) Proprietor: Adaptive Biotechnologies Corporation, Seattle, Washington 98102 (US)
(72) Inventor: PEPIN, Francois, South San Francisco, CA 94080 (US); FAHAM, Malek, South San Francisco, CA 94080 (US); MOORHEAD, Martin, South San Francisco, CA 94080 (US)
(74) Representative: Boult Wade Tennant LLP
(86) International application number: PCT/US2014/017416
(87) International publication number: WO 2014/130685

(56) References cited:
- WO-A1-2011/139371
- US-A1- 2013 196 328
- WU DAVID ET AL: "High-throughput sequencing detects minimal residual disease in acute T lymphoblastic leukemia", SCIENCE TRANSLATIONAL MEDICINE, AAAS - AMERICAN ASSOCIATION FOR THE ADVANCEMENT OF SCIENCE, US, vol. 4, no. 134, 16 May 2012 (2012-05-16), pages 151-157, XP008164047, ISSN: 1946-6242, DOI: 10.1126/SCITRANSLMED.3003656
- C. GAWAD ET AL: "Massive evolution of the immunoglobulin heavy chain locus in children with B precursor acute lymphoblastic leukemia", BLOOD, vol. 120, no. 22, 28 August 2012 (2012-08-28), pages 4407-4417, XP055066136, ISSN: 0006-4971, DOI: 10.1182/blood-2012-05-429811
- R. WINCHESTER ET AL: "Circulating Activated and Effector Memory T Cells Are Associated with Calcification and Clonal Expansions in Bicuspid and Tricuspid Valves of Calcific Aortic Stenosis", THE JOURNAL OF IMMUNOLOGY, vol. 187, no. 2, 15 July 2011 (2011-07-15) , pages 1006-1014, XP055303430, US ISSN: 0022-1767, DOI: 10.4049/jimmunol.1003521
- WOODSWORTH DANIEL J ET AL: "Sequence analysis of T-cell repertoires in health and disease.", GENOME MEDICINE 2013, vol. 5, no. 10, 30 October 2013 (2013-10-30), page 98, XP055186034, ISSN: 1756-994X

## Description

### BACKGROUND OF THE INVENTION

Large-scale DNA sequencing in diagnostic and prognostic applications has expanded rapidly as its speed has increased and its per-base cost has decreased, e.g. Ding et al, Nature, 481(7382): 506-510 (2012); Chiu et al, Brit. Med. J., 342: c7401 (2011); Ku et al, Annals of Neurology, 71(1): 5-14 (2012); and the like. In particular, profiles of nucleic acids encoding immune molecules, such as T cell or B cell receptors, or their components, contain a wealth of information on the state of health or disease of an organism, so that the use of such profiles as diagnostic or prognostic indicators has been proposed for a wide variety of conditions, e.g. Faham and Willis, U.S. patent publication 2010/0151471; Freeman et al, Genome Research, 19: 1817-1824 (2009); Boyd et al, Sci. Transl. Med., 1(12): 12ra23 (2009); He et al, Oncotarget (March 8, 2011).

Patients treated for many cancers often retain a minimal residual disease (MRD) related to the cancer. That is, even though a patient may have by clinical measures a complete remission of the disease in response to treatment, a small fraction of the cancer cells may remain that have, for one reason or another, escaped destruction. The type and size of this residual population is an important prognostic factor for the patient's continued treatment, e.g. Campana, Hematol. Oncol. Clin. North Am., 23(5): 1083-1098 (2009); Buccisano et al, Blood, 119(2): 332-341 (2012). Consequently, several techniques for assessing this population have been developed, including techniques based on flow cytometry, in situ hybridization, cytogenetics, amplification of nucleic acid markers, and the like, e.g. Buccisano et al, Current Opinion in Oncology, 21: 582-588 (2009); van Dongen et al, Leukemia, 17(12): 2257-2317 (2003); and the like. The amplification of nucleic acids encoding segments of recombined immune receptors (i.e. clonotypes) have been particularly useful in assessing MRD in leukemias and lymphomas, since such clonotypes typically have unique sequences which may serve as molecular tags for their associated cancer cells. However, not all clonotypes encode highly diverse receptor segments, such as V(D)J segments. Clonotypes not infrequently encode receptor segments of lower diversity, such as DJ segments, or segments that form recurrent motifs, preferentially represented for possible developmental or functional reasons, and thereby being relatively common among different individuals, e.g. Gauss et al, Mol. Cell. Biol., 16(1): 258-269 (1996); Murugan et al, Proc. Natl. Acad. Sci., 109(40): 16161-16166 (2012); Venturi et al, J. Immunol., 186: 4285-4294 (2011); Robins, J. Immunol., 189(6): 3221-3230 (2012). In either circumstance, such clonotypes may be suboptimal, or even fail, as markers for assessing MRD.

In view of the foregoing, it would be highly advantageous if a method were available for assessing clonotypes for rarity or uniqueness in order to determine whether they are likely to provide an accurate measure of minimal residual disease, with a low likelihood of false positive outcomes.

### SUMMARY OF THE INVENTION

The present invention is directed to a method of selecting rare clonotypes for monitoring minimal residual disease of a cancer in order to minimize false positive outcomes. The invention is defined by the appended set of claims.

Disclosed herein is a method of selecting one or more patient-specific clonotypes correlated with a lymphoid proliferative disorder for monitoring a minimal residual disease thereof, the method comprising the following steps: (a) obtaining a diagnostic sample from the patient comprising T-cells and/or B-cells; (b) amplifying molecules of nucleic acid from the T-cells and/or B-cells of the sample, the molecules of nucleic acid comprising recombined DNA sequences from T-cell receptor genes or immunoglobulin genes; (c) sequencing the amplified molecules of nucleic acid to form a clonotype profile; (d) selecting a set of one or more candidate clonotypes based on their frequency in the clonotype profile; (e) comparing the one or more candidate clonotypes from the set to clonotypes of a clonotype database containing clonotypes from at least one individual other than the patient to determine a presence, absence and/or level in the clonotype database of each candidate clonotype and removing from the set any candidate clonotype having a frequency in the clonotype database greater than a predetermined frequency; and (f) selecting as the one or more patient-specific clonotypes for monitoring the minimal residual disease the candidate clonotypes remaining in the set.

Further disclosed is a method of selecting one or more patient-specific clonotypes correlated with a lymphoid neoplasm for monitoring a minimal residual disease thereof, wherein the method comprising the steps of: (a) obtaining a sample from the patient comprising T-cells and/or B-cells; (b) amplifying molecules of nucleic acid from the T-cells and/or B-cells of the sample, the molecules of nucleic acid comprising recombined DNA sequences from T-cell receptor genes or immunoglobulin genes; (c) sequencing the amplified molecules of nucleic acid to form a clonotype profile; (d) comparing clonotypes from the clonotype profile to clonotypes of a clonotype database containing clonotypes from at least one individual other than the patient to determine a presence, absence and/or level in the clonotype database of each clonotype from the clonotype profile; and (e) selecting the one or more patient-specific clonotypes for monitoring the minimal residual disease which are each correlated with the lymphoid neoplasm and which are each absent from the clonotype database or at a level in the clonotype database below a predetermined frequency.

Further disclosed is a method for determining whether a tissue sample comprising T cells and/or B cells from a first individual contaminates a tissue sample comprising T cells and/or B cells from a second individual, wherein the method comprising the steps of: (a) generating a first clonotype profile from nucleic acid of the tissue sample from the first individual; (b) generating a second clonotype profile from nucleic acid of the tissue sample from the second individual; (c) comparing clonotypes from the first and second clonotype profiles to clonotypes of a clonotype database containing clonotypes from at least one individual other than the first and second individual to determine a presence, absence and/or level in the clonotype database of each clonotype from the first and second clonotype profiles which are each absent from the clonotype database or at a level in the clonotype database below a predetermined threshold; and (d) classifying the tissue sample from the first individual as contaminated by nucleic acids from the second individual whenever any of such determined clonotypes are present in both the first and second clonotype profiles.

### BRIEF DESCRIPTION OF THE DRAWINGS

The novel features of the invention are set forth with particularity in the appended claims. A better understanding of the features and advantages of the present invention is obtained by reference to the following detailed description that sets forth illustrative embodiments, in which the principles of the invention are utilized, and the accompanying drawings of which:
Figs. 1A-1C show a two-staged PCR scheme for amplifying and sequencing IgH or TCRβ genes.
Figs. 2A-2B illustrate different embodiments for determining a clonotype based on sequence reads of an amplicon produced by the method illustrated in Figs. 1A-1C.
FIG. 3A illustrates a PCR scheme for generating three sequencing templates from an IgH chain in a single reaction. Figs. 3B-3C illustrates a PCR scheme for generating three sequencing templates from an IgH chain in three separate reactions after which the resulting amplicons are combined for a secondary PCR to add P5 and P7 primer binding sites. Fig. 3D illustrates the locations of sequence reads generated for an IgH chain.
Figs. 4A-4B illustrates a clonotype frequency model that may be employed to summarize information in a clonotype database useful in selecting rare clonotypes.

### DETAILED DESCRIPTION OF THE INVENTION

The practice of the present invention may employ, unless otherwise indicated, conventional techniques and descriptions of molecular biology (including recombinant techniques), bioinformatics, cell biology, and biochemistry, which are within the skill of the art. Such conventional techniques include, but are not limited to, sampling and analysis of blood cells, nucleic acid sequencing and analysis, and the like. Specific illustrations of suitable techniques can be had by reference to the example herein below. However, other equivalent conventional procedures can, of course, also be used. Such conventional techniques and descriptions can be found in standard laboratory manuals such as Genome Analysis: A Laboratory Manual Series (Vols. I-IV); PCR Primer: A Laboratory Manual*; and* Molecular Cloning: A Laboratory Manual (all from Cold Spring Harbor Laboratory Press); and the like.

Disclosed herein are methods for selecting a rare clonotype, and/or a rare group of related clonotypes, which are correlated with a lymphoid or myeloid proliferative disorder and which can be used to monitor the status of the disorder with minimum likelihood that a false positive determination will be made of disease recurrence. Such rare clonotypes are particularly useful for monitoring minimal residual disease of a cancer after treatment, where the result of such monitoring is a key factor in determining whether to continue, discontinue or otherwise modify treatment. In many malignant lymphoid and myeloid neoplasms, a diagnostic tissue sample (or also referred to herein as a "diagnostic sample") such as a peripheral blood sample or a bone marrow sample, is obtained before treatment from which a clonotype profile is generated (a "diagnostic clonotype profile"). One or more disease-correlated clonotypes (i.e. "correlating clonotypes") are identified in the clonotype profile, usually as the clonotypes having the highest frequencies. For example, a predetermined diagnostic frequency may be determined and every clonotype having a frequency greater than the predetermined diagnostic frequency is selected as a member of a set of candidate clonotypes. In some embodiments, one or more correlating clonotypes used to monitor MRD are taken from the set of candidate clonotypes that are sufficiently rare so as to minimize false positive determinations of MRD. The number of candidate clonotypes in a set may vary depending on the type of cancer, the stage of cancer, treatment history, and the like. In some embodiments, a set includes up to 10 of the highest frequency clonotypes of a diagnostic clonotype profile. In other embodiments, a set includes up to 5 of the highest frequency clonotypes of a diagnostic clonotype profile. After treatment, and preferably after attainment of a complete remission of the cancer, the presense, absence or frequency of such correlating clonotypes is assessed periodically to determine whether the remission is holding or whether the neoplasm is returning or relapsing, based on the presence of, or an increase in the frequency of, the correlating clonotypes (or related clonotypes) in a post-treatment clonotype profile. That is, after treatment, minimal residual disease of the cancer is assessed based on the presence, absence or frequency of the correlating clonotypes. Usually, if the frequency or level of such a correlating clonotype increases to equal or exceed a predetermined value, e.g., .01 percent, or 0.1 percent, or 1 percent (which may depend on the disease, the stage of the disease, the patient's condidtion, or the like), then a treatment decision may be made, such as, to implement further treatment in accordance with a previous protocol, to modify a treatment protocol, such as by increasing dosages, adding different treatment modalities, such as using a different drug, or the like. As mentioned above, when such correlating clonotypes are common or correspond to a rearranged receptor segment that lacks sufficient diversity (so that non-cancerous cells may share the clonotype), the occurrence of such clonotypes in a post-treatment clonotype profile may give rise to a false positive indication of relapse. Further disclosed is a method for assessing the rarity of correlating clonotypes, and therefore, a method for selecting correlating clonotypes that minimize the likelihood of false positive determinations of relapse. In this aspect, after a diagnostic clonotype profile is generated, a clonotype database is searched for the presence of both identical clonotypes and clonotypes related by clan, as the concept is described more fully below. In this manner, regardless of which correlating clonothypes are selected for monitoring, even before post-treatment testing begins (whether by clonotype profiling or direct PCR of the selected clonotype(s)), a likelihood that the selected clonotypes may give rise to a false positive indication may be estimated.

Methods disclosed herein are applicable to monitoring any proliferative disease in which a rearranged nucleic acid encoding an immune receptor or portion thereof can be used as a marker of cells involved in the disease. Thus the method is applicable to both sequence-based methods involving the generation of clonotype profiles in each post-treatment measurement as well as single-clone tracking methods involving a PCR amplification of a single clonotype. In one aspect, methods disclosed herein are applicable to lymphoid and myeloid proliferative disorders. In another aspect, the methods are applicable to lymphomas and leukemias. In another aspect, the methods are applicable to monitoring MRD in follicular lymphoma, chronic lymphocytic leukemia (CLL), acute lymphocytic leukemia (ALL), chronic myelogenous leukemia (CML), acute myelogenous leukemia (AML), Hodgkins's and non-Hodgkin's lymphomas, multiple myeloma (MM), monoclonal gammopathy of undetermined significance (MGUS), mantle cell lymphoma (MCL), diffuse large B cell lymphoma (DLBCL), myelodysplastic syndromes (MDS), T cell lymphoma, or the like. In a particular embodiment, a method disclosed herein is particularly well suited for monitoring MRD in ALL, MM or DLBCL.

In some embodiments, particularly for lymphoid and myeloid proliferative disorders, a plurality of clonotype profiles are generated from a diagnostic sample so that multiple types of clonotypes may be assessed. That is, recombined DNA or nucleic acid sequences (also referred to herein as "somatically rearranged DNA or nucleic acids" or "rearranged DNA or nucleic acid") from T-cells and/or B-cells may include nucleic acid sequences that encode full chains or portions of the following: a VDJ rearrangement of IgH, a DJ rearrangement of IgH, a VJ rearrangement of IgK, a VJ rearrangement of IgL, a VDJ rearrangement of TCR β, a DJ rearrangement of TCR β, a VJ rearrangement of TCR α, a VJ rearrangement of TCR γ, a VDJ rearrangement of TCR δ, and a VD rearrangement of TCR δ. If it is known through other tests that a patient's cancer is derived from B-cells, the clonotypes analyzed in a diagnostic sample may be limited to nucleic acid sequences that encode full chains or portions of the following: a VDJ rearrangement of IgH, a DJ rearrangement of IgH, a VJ rearrangement of IgK, and/or a VJ rearrangement of IgL. Likewise, if it is known through other tests that a patient's cancer is derived from T-cells, the clonotypes analyzed in a diagnostic sample may be limited to nucleic acid sequences that encode full chains or portions of the following: a VDJ rearrangement of TCR β, a DJ rearrangement of TCR β, a VJ rearrangement of TCR α, a VJ rearrangement of TCR γ, a VDJ rearrangement of TCR δ, and/or a VD rearrangement of TCR δ. In some embodiments, clonotype profiles generated from a diagnostic sample in the method disclosed herein are based on clonotypes encoding full chains or portions of the following: a VDJ-containing segment of IgH, a DJ-containing segment of IgH, a VJ-containing segment of IgK, a VDJ-containing segment of TCR β, a segment of TCR γ, and/or a segment of TCR δ. In some embodiments, diagnostic samples may comprise tissues from bone marrow, peripheral blood or lymph nodes or other lymphoid tissue.

In some embodiments, the above aspect may be implemented with the following steps: (a) obtaining a diagnostic sample from the patient comprising T-cells and/or B-cells; (b) amplifying molecules of nucleic acid from the T-cells and/or B-cells of the sample, the molecules of nucleic acid comprising recombined DNA sequences from T-cell receptor genes or immunoglobulin genes; (c) sequencing the amplified molecules of nucleic acid to form a clonotype profile; (d) selecting a set of one or more candidate clonotypes based on their frequency in the clonotype profile; (e) comparing the one or more candidate clonotypes from the set to clonotypes of a clonotype database containing clonotypes from at least one individual other than the patient to determine a presence, absence and/or level in the clonotype database of each candidate clonotype and removing from the set any candidate clonotype having a frequency in the clonotype database greater than a predetermined frequency; and (f) selecting as the one or more patient-specific clonotypes for monitoring the minimal residual disease the candidate clonotypes remaining in the set. As discussed more fully below, the step of comparing candidate clonotypes of a set obtained from the clonotype profile (i.e. "measured clonotypes") with those in the clonotype database is carried out by searching the clonotype database for matching clonotypes or clonotypes that otherwise are related, for example, by closeness of sequence or by clonal evolutions. In some embodiments, the step of comparing may be implemented by requiring exact matching between measured clonotypes and database clonotypes in order to count as match; in other embodiments, the step of comparing may be implemented by requiring matching of only a subset of nucleotides between measured clonotypes and database clonotypes in order to count as match. For example, in some embodiments, a database clonotype may be counted as matching a measured clonotype if there is greater than 90 percent identity between the two. In some embodiments, a database clonotype may be counted as matching a measured clonotype if it is related by being in the same clan as the measure clonotype. In some embodiments, a clonotype may be useful as a clonotype to monitor MRD or contamination, even though it is present in a clonotype database at some low, predetermined threshold or frequency. In such embodiments, a clonotype may be employed to monitor MRD or detect contamination if it is present at or below a predetermined frequency, which may depend on the size of, or the number of clonotype entries in, the database. In one embodiment, a predetermined frequency is 10⁻⁷; in another embodiment, a predetermined frequency is 10⁻⁸; and in another embodiment; a predetermined frequency is 10⁻⁹.

In some embodiments, a candidate clonotype may be removed from a set whenever a sequence difference between it and a member of a clonotype database is below some minimum value (referred to herein as a "predetermined difference"). Sequence differences include base substitution, insertion and deletion differences which may be measure by a sequence distance measure, such as a Hamming distance. Sequence differences also include differences due to somatic rearrangements, such as, VH replacements. In some embodiments, a candidate clonotype may be removed from a set if its sequence is within some predetermined sequence distance measure of a clonotype sequence in the database. In one embodiment, such sequence distance measure is a Hamming distance.

In the event that no candidate clonotypes remain in a set after application of the steps of the disclosed method, choices for the predetermined diagnostic frequency for selecting candidate clonotypes or for the predetermined frequency for rejecting candidate clonotypes from a set may be altered to obtain correlating clonotypes for MRD monitoring that have a higher likelihood of false positive result. In the event that a plurality of candidate clonotypes remain in a set after application of the steps of the invention, one or more of the remaining members may be used to monitor MRD, e.g. as taught by Faham and Willis, e.g. U.S. patents 8,236,503 or 8,628,927. In some embodiments, a single candidate clonotype that has the greatest difference from the database clonotypes may be used to monitor MRD. In some embodiments, the predetermined frequency and predetermined diagnostic frequency are selected so that at least one candidate clonotype remains in the set after application of the steps of the disclosed method.

In regard to counting two clonotypes as "matched" based on clan membership, in some embodiments, two clonotypes may be deemed to be in the same clan if one or more of the following criteria are met: (a) clonotypes are at least ninety percent identical to one another; (b) clonotypes comprise recombined sequences from immunoglobulin heavy chain that are related by a VH replacement; (c) clonotypes comprise recombined sequences from immunoglobulin heavy chain that are related by hypermutation; and (d) clonotypes are related in that they have identically mutated V region and J region but have a different NDN region.

Further disclosed is a method of selecting rare clonotypes and/or rare groups of related clonotypes, or clans, which can be used to detected and/or quantify cross sample contamination, particularly among samples in which nucleotide sequences encoding rearranged immune receptors are amplified by techniques such as PCR. In settings, such as a clinical laboratory, where samples from different patients are processed by generating clonotype profiles as described herein, there is a significant probability of cross contamination of samples, which may involve the recording of clonotypes derived from a first sample as being members of a clonotype profile of a second sample. Such potential contamination may be rapidly detected by comparing rare clonotypes of a clonotype profile from the first sample with those of a clonotype profile from the second sample. If one or more matches are present, then cross-contamination between the samples is highly likely.

In some embodiments, the above aspect may be implemented with the following steps: (a) generating a first clonotype profile from nucleic acid of the tissue sample from the first individual; (b) generating a second clonotype profile from nucleic acid of the tissue sample from the second individual; (c) comparing clonotypes from the first and second clonotype profiles to clonotypes of a clonotype database containing clonotypes from at least one individual other than the first and second individual to determine a presence, absence and/or level in the clonotype database of each clonotype from the first and second clonotype profiles which are each absent from the clonotype database or at a level in the clonotype database below a predetermined threshold; and (d) classifying the tissue sample from the first individual as contaminated by nucleic acids from the second individual whenever any of such determined clonotypes are present in both the first and second clonotype profiles.

In some embodiments, a method for generating clonotype profiles of recombined DNA sequences in T-cells and/or B-cells comprises the following steps: (a) obtaining a sample from a subject comprising T-cells and/or B-cells; (b) spatially isolating individual molecules of recombined DNA sequences from said cells on a solid substrate; (c) sequencing said spatially isolated individual molecules of recombined DNA sequences to provide at least 1000 sequence reads each having an error rate; (d) coalescing sequence reads into different recombined DNA sequences of the sample whenever the sequence reads are distinct with a confidence of at least 99.9 percent; and (e) determining the levels of the different recombined DNA sequences from said sample to generate said profile of recombined DNA sequences.

In some embodiments, a clonotype database may be any database of clonotypes containing clonotypes from at least one individual other than the patient. Usually, the clonotype database is a collection of clonotypes from clonotype profiles of a plurality of individuals. For example, such collection may include clonotype profiles of at least 10⁴ clonotypes each from at least 10 individuals other than the patient; or such collection may include clonotype profiles of at least 10⁴ clonotypes each from at least 100 individuals. In another embodiment, a clonotype database comprises clonotype profiles each having at least 10⁴ clonotypes from a population of individuals, so that the database contains at least 10⁸ clonotypes, or 10⁹ clonotypes, or 10¹⁰ clonotypes. The foregoing clonotype databases may comprise clonotypes that encode specific segments of particular immune receptor molecules. As discussed more fully below, in one aspect, clonotypes obtained in a method disclosed herein encode segments of immune receptor molecules that are identical or substantially identical to those encoded by clonotypes of the clonotype database searched in the method, where "substantially identical" means that there is sufficient overlap so that measured clonotypes may be effectively searched against database clonotypes. In one embodiment, clonotypes obtained in the method and database clonotypes both encode a V(D)J region of an IgH molecule or a portion thereof; in another embodiment, clonotypes obtained in the method and database clonotypes both encode a DJ region of an IgH molecule or a portion thereof; in another embodiment, clonotypes obtained in the method and database clonotypes both encode an IgK molecule or a portion thereof; in another embodiment, clonotypes obtained in the method and database clonotypes both encode a TCRβ molecule or a portion thereof; in another embodiment, clonotypes obtained in the method and database clonotypes both encode a TCRγ molecule or a portion thereof; in another embodiment, clonotypes obtained in the method and database clonotypes both encode a TCRδ molecule or a portion thereof. In other embodiments, measured clonotypes and database clonotypes both encode segments of the following immune receptors: a VDJ rearrangement of IgH, a DJ rearrangement of IgH, a VJ rearrangement of IgK, a VJ rearrangement of IgL, a VDJ rearrangement of TCR β, a DJ rearrangement of TCR β, a VJ rearrangement of TCR α, a VJ rearrangement of TCR γ, a VDJ rearrangement of TCR δ, and a VD rearrangement of TCR δ In some embodiments, measured clonotypes and database clonotypes both have lengths in the range of from 25 to 400 nucleotides. In some embodiments, measured clonotypes and database clonotypes have the same lengths.

In some embodiments, individuals contributing to a clonotype database may be from a related population or group. For example, a clonotype databases may be specific for individuals that have a particular disease, e.g. a lymphoid or myeloid proliferative disorder, lymphoid neoplasm, or a constituent condition, e.g. MGUS. In some embodiments, individuals contributing to a clonotype database may be from a related ethnic or racial group, such as, African, Japanese, Caucasian, or the like. In one embodiment, a clonotype database comprises clonotype profiles each have at least 10⁴ clonotypes obtained from a plurality of patients suffering from B-cell neoplasms. In another embodiment, a clonotype database comprises clonotype profiles each have at least 10⁴ clonotypes obtained from plurality of patients suffering from B-cell lymphomas. In another embodiment, a clonotype database comprises clonotype profiles each have at least 10⁴ clonotypes obtained from a plurality of patients suffering from diffuse large B-cell lymphomas. In another embodiment, a clonotype database comprises clonotype profiles each have at least 10⁴ clonotypes obtained from a plurality of patients suffering from multiple myeloma.

In another embodiment, a clonotype database comprises clonotype profiles each have at least 10⁴ clonotypes obtained from a plurality patients suffering from T-cell neoplasms.

A wide variety of search methods or algorithms may be used to carry out the step of comparing measured clonotypes to database clonotypes. Many conventional sequence alignment and searching algorithms are publicly available and have been described in the following references: Mount, Bioinformatics Sequence and Genome Analysis, Second Edition (Cold Spring Harbor Press, 2004); Batzoglou, Briefings in Bioinformatics, 6: 6-22 (2005); Altschul et al, J. Mol. Biol., 215(3): 403-410 (1990); Needleman and Wunsch, J. Mol. Biol., 48: 443-453 (1970); Smith and Waterman, Advances in Applied Mathematics, 2: 482-489 (1981); and the like. As discussed more fully below, some steps of comparing may require more specialized searching methods, which may be implemented by those having ordinary skill in the field of bioinformatics; for example, for searching clonotypes related by rearrangements, such as VH replacements, or the like, may require obvious modifications of available search methods or algorithms. For example, it may not make sense to conduct a conventional alignment between a VDJ-based clonotype and those in a database in order to determine the presence of VH replacements. One of ordinary skill in the art would understand that, in such a case, an alignment could be conducted using the clonotype sequence less the V region sequences.

In one aspect, clonotype databases are searched not only for clonotypes identical to measured clonotypes, but also for clonotypes that are related, for example, by being members of the same clan, or by having a phylogenic relationship. Thus, in some embodiments, a search of a clonotype database will retrieve any database clonotype that is a member of the same clan as the measured clonotype. Such retrieval indicates the presence of a clan member which may or may not have a sequence identical to the measured clonotype, but which satisfies one or more relatedness criterion for determining clan membership. Exemplary criteria for defining a clanmay include one or more of the following: (a) clonotypes are at least ninety percent identical to each other, (b) clonotypes encode IgH segments and are identical except for different mutations from somatic hypermutation, (c) clonotypes are related by a VH replacement, (d) clonotypes have identical V regions and identical J regions including identical mutations in each region, but have different NDN regions, (e) clonotypes have identical sequences, except for one or more insertions and/or deletions of from 1-10 bases. In some embodiments, in the foregoing example (e), clonotypes may be member of the same clan if they have identical sequences, except for one or more insertions and/or deletions of from 1-5 bases, or from 1-3 bases.

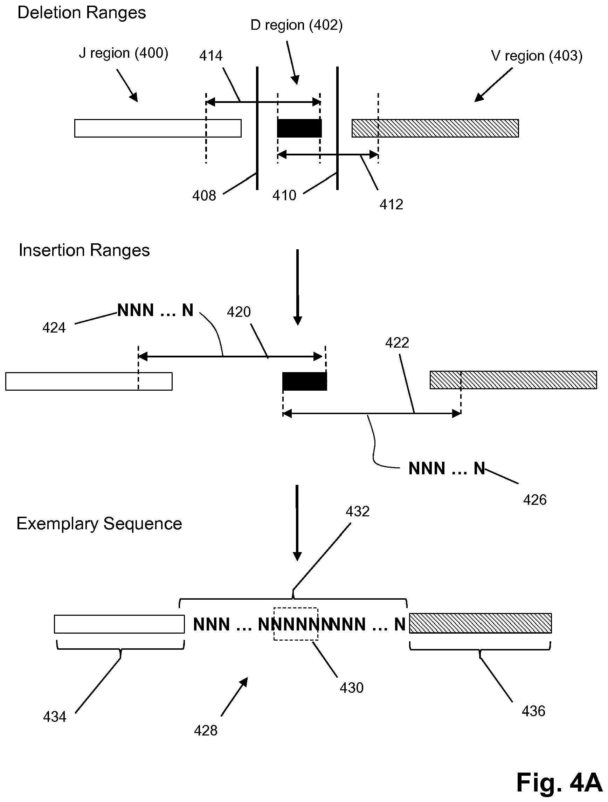

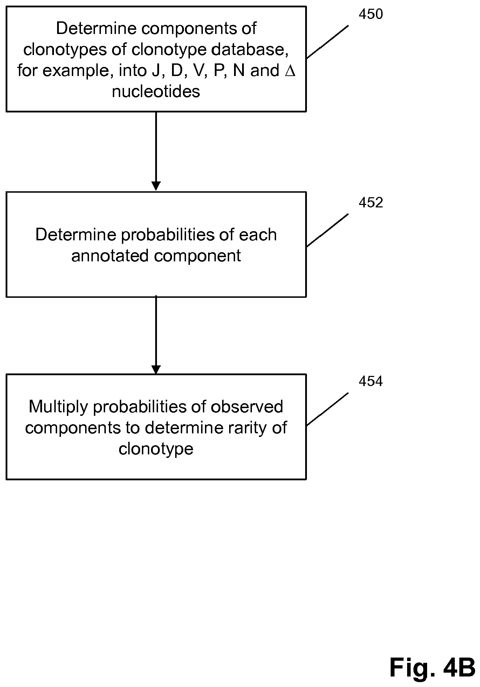

In another aspect, information from one or more clonotype databases may be used to determine parameters of a model of clonotype development, which can then be used to rapidly provide frequencies, or probabilities of occurrence, of clonotypes based on their structure, that is, the types and/or sizes of their constituent gene segments and their modifications by the rearrangement process. For example, Murugan et al (cited above) provides an example of such a model for a limited set of clonotypes. Another example is illustrated with the help of Fig. 4A for the case of clonotypes encoding VDJ regions of IgH or TCRβ molecules. Examination of clonotype databases has indicated that not every clonotype that can possibly be generated by natural processes is equally probable. The process for generating diversity of a VDJ region is illustrated in Fig. 4A. Roughly the process involves mechanisms for (i) adding "P" nucleotides to the ends of D and J gene segments, (ii) deleting nucleotides from J or D gene segments at the DJ junction and from D or V gene segments at the DV junction, and (iii) inserting randomly from 1-40 nucleotides at each such junction, e.g. Janeway et al, Immunobiology, Sixth Edition (Garland Science, New York, 2005). Thus all the possible outcomes of this process may be listed and their respective frequencies determined from clonotype databases. Alternatively, the frequencies of the component segments may be determined, such as the frequence of a J region (or a J region with a given truncation), a V region (or a V region with a given truncation), an NDN region, or so on. Such frequency models may be used in lieu of a search of a clonotype database to determine the rarity of a particular measured clonotype. This concept is outlined in the flow chart of Fig. 4B. A clonotype (e.g. a VDJ recombinant) is made by operation of one or more of the above processes. Since the processes are probabilistic and work at cross purposes in some cases (e.g. nucleotide additions versus nucleotide deletions), almost every clonotype may be produced by multiple pathways through the above operations (for example, adding 9 nucleotides then deleting 6 nucleotides (for a net addition of 3) may be equivalent to adding 4 then deleting 1). However, the probability of adding 9 followed by deleting 6 may have a very different probability than that of adding 4 followed by deleting 1. A clonotype database may be used to estimate the probabilities associated with the various operations that result in given clonotypes. That is, a clonotype database may be used to annotate each clonotype characteristic with a probability of its occurrence (452). As noted in (450) of Fig. 4B, for a clonotype based on a VDJ rearrangement, such characteristics may include J segment selection, D segment selection, V segment selection, P nucleotides present, N nucleotide added, Δ (number of nucleotides deleted), and the like. Once such a model is obtained, then the rarity (or estimate of frequency) of a given clonotype based on its component characteristics may be determined by multiplying the component probabilities together (454).

Returning to Fig. 4A, an IgH or TCRβ molecule comprises a VDJ region assembled from J region (400), D region (402) and V region (403); however, during assembly 0 to several nucleotides at the ends of the gene segments at the JD junction and DV junction are deleted. If lines (408) and (410) indicate the positions of the JD and DV junctions absence deletions, the ranges (414) and (412) indicated by arrows, illustrates the range of possible deletions from the ends of the gene segments. In some cases, D region (402) may be completely deleted. Diversity is further generated by a mechanism that inserts 0 to about 20 nucleotides at each junction. Thus after deletions, oligonucleotide segments (424) and (426) are inserted between the (possibly truncated) ends of the gene segments. The positioning of the insertion depends on what was deleted, but it occurs within ranges (420) and (422). As a result of these mechanisms a very large number of possible clonotypes (428) are generated that may or may not include a recognizable D region (430). The likelihoods of any of these rearrangements may be estimated by examining their occurrences in one or more clonotype databases. Thus, J region (434) of a particular type and having a particular length may have an expected frequency of occurrence; likewise, NDN region (432) of a particular length and presence of absence of an identifiable D gene segment may have an expected frequency of occurrence; and so on for V region (436). In one embodiment of this aspect, the rarity of a measured clonotype may be quickly estimated by multiplying the expected frequencies (or probabilities) of its constituent regions.

### Samples

Clonotype profiles may be obtained from samples of immune cells. For example, immune cells can include T-cells and/or B-cells. T-cells (T lymphocytes) include, for example, cells that express T cell receptors. T-cells include helper T cells (effector T cells or Th cells), cytotoxic T cells (CTLs), memory T cells, and regulatory T cells. In one aspect a sample of T cells includes at least 1,000T cells; but more typically, a sample includes at least 10,000 T cells, and more typically, at least 100,000 T cells. In another aspect, a sample includes a number of T cells in the range of from 1000 to 1,000,000 cells. A sample of immune cells may also comprise B cells. B-cells include, for example, plasma B cells, memory B cells, B1 cells, B2 cells, marginal-zone B cells, and follicular B cells. B-cells can express immunoglobulins (antibodies, B cell receptor). As above, in one aspect a sample of B cells includes at least 1,000 B cells; but more typically, a sample includes at least 10,000 B cells, and more typically, at least 100,000 B cells. In another aspect, a sample includes a number of B cells in the range of from 1000 to 1,000,000 B cells.

Samples used in the methods of the invention can come from a variety of tissues, including, for example, tumor tissue, blood and blood plasma, lymph fluid, cerebrospinal fluid surrounding the brain and the spinal cord, synovial fluid surrounding bone joints, and the like. In one embodiment, the sample is a blood sample. The blood sample can be about 0.1, 0.2, 0.3, 0.4, 0.5, 0.6, 0.7, 0.8, 0.9, 1.0, 1.5, 2.0, 2.5, 3.0, 3.5, 4.0, 4.5, or 5.0 mL. The sample can be a tumor biopsy. The biopsy can be from, for example, from a tumor of the brain, liver, lung, heart, colon, kidney, or bone marrow. Any biopsy technique used by those skilled in the art can be used for isolating a sample from a subject. For example, a biopsy can be an open biopsy, in which general anesthesia is used. The biopsy can be a closed biopsy, in which a smaller cut is made than in an open biopsy. The biopsy can be a core or incisional biopsy, in which part of the tissue is removed. The biopsy can be an excisional biopsy, in which attempts to remove an entire lesion are made. The biopsy can be a fine needle aspiration biopsy, in which a sample of tissue or fluid is removed with a needle.

The sample can be a biopsy, e.g., a skin biopsy. The biopsy can be from, for example, brain, liver, lung, heart, colon, kidney, or bone marrow. Any biopsy technique used by those skilled in the art can be used for isolating a sample from a subject. For example, a biopsy can be an open biopsy, in which general anesthesia is used. The biopsy can be a closed biopsy, in which a smaller cut is made than in an open biopsy. The biopsy can be a core or incisional biopsy, in which part of the tissue is removed. The biopsy can be an excisional biopsy, in which attempts to remove an entire lesion are made. The biopsy can be a fine needle aspiration biopsy, in which a sample of tissue or fluid is removed with a needle.

The sample can be obtained from bodily material which is left behind by a subject. Such discarded material can include human waste. Discarded material could also include shed skin cells, blood, teeth or hair.

In the case of lymphoproliferative disorders, such as lymphomas, a calibrating or diagnostic sample may be obtained from lymphoid tissues, from lesions caused by the disorder, e.g. metastatic lesions, or from tissues indirectly affected by the disorder. For lymphoid neoplasms there is widely available guidance and commercially available kits for immunophenotyping and enriching disease-related lymphocytes, e.g. "U.S.-Canadian consensus recommendations on the immunophenotypic analysis of haematologic neoplasia by flow cytometry," Cytometry, 30: 214-263 (1997); MultiMix™ Antibody Panels for Immunophenotyping Leukemia and Lymphoma by Flow Cytometry (Dako, Denmark); and the like. Lymphoid tissues include lymph nodes, spleen, tonsils, adenoids, thymus, and the like.

The sample can include nucleic acid, for example, DNA (e.g., genomic DNA) or RNA (e.g., messenger RNA). The nucleic acid can be cell-free DNA or RNA, e.g. extracted from the circulatory system, Vlassov et al, Curr. Mol. Med., 10: 142-165 (2010); Swarup et al, FEBS Lett., 581: 795-799 (2007). In the methods disclosed herein, the amount of RNA or DNA from a subject that can be analyzed includes, for example, as low as a single cell in some applications (e.g., a calibration test) and as many as 10 million of cells or more translating to a range of DNA of 6pg-60ug, and RNA of approximately 1pg-10ug.

In one aspect, a sample of lymphocytes for generating a clonotype profile is sufficiently large that substantially every T cell or B cell with a distinct clonotype is represented therein. In one embodiment, a sample is taken that contains with a probability of ninety-nine percent every clonotype of a population present at a frequency of .001 percent or greater. In another embodiment, a sample is taken that contains with a probability of ninety-nine percent every clonotype of a population present at a frequency of .0001 percent or greater. In one embodiment, a sample of B cells or T cells includes at least a half million cells, and in another embodiment such sample includes at least one million cells.

Whenever a source of material from which a sample is taken is scarce, such as, clinical study samples, or the like, DNA from the material may be amplified by a non-biasing technique, such as whole genome amplification (WGA), multiple displacement amplification (MDA); or like technique, e.g. Hawkins et al, Curr. Opin. Biotech., 13: 65-67 (2002); Dean et al, Genome Research, 11: 1095-1099 (2001); Wang et al, Nucleic Acids Research, 32: e76 (2004); Hosono et al, Genome Research, 13: 954-964 (2003); and the like.

Blood samples are of particular interest and may be obtained using conventional techniques, e.g. Innis et al, editors, PCR Protocols (Academic Press, 1990); or the like. For example, white blood cells may be separated from blood samples using convention techniques, e.g. RosetteSep kit (Stem Cell Technologies, Vancouver, Canada). Blood samples may range in volume from 100 µL to 10 mL; in one aspect, blood sample volumes are in the range of from 100 µL to 2 mL. DNA and/or RNA may then be extracted from such blood sample using conventional techniques for use in methods disclosed herein, e.g. DNeasy Blood & Tissue Kit (Qiagen, Valencia, CA). Optionally, subsets of white blood cells, e.g. lymphocytes, may be further isolated using conventional techniques, e.g. fluorescently activated cell sorting (FACS)(Becton Dickinson, San Jose, CA), magnetically activated cell sorting (MACS)(Miltenyi Biotec, Auburn, CA), or the like.

Since the identifying recombinations are present in the DNA of each individual's adaptive immunity cells as well as their associated RNA transcripts, either RNA or DNA can be sequenced in the methods disclosed herein. A recombined sequence from a T-cell or B-cell encoding a T cell receptor or immunoglobulin molecule, or a portion thereof, is referred to as a clonotype. The DNA or RNA can correspond to sequences from T-cell receptor (TCR) genes or immunoglobulin (Ig) genes that encode antibodies. For example, the DNA and RNA can correspond to sequences encoding α, β, γ, or δ chains of a TCR. In a majority of T-cells, the TCR is a heterodimer consisting of an α-chain and β-chain. The TCRα chain is generated by VJ recombination, and the β chain receptor is generated by V(D)J recombination. For the TCRβ chain, in humans there are 48 V segments, 2 D segments, and 13 J segments. Several bases may be deleted and others added (called N and P nucleotides) at each of the two junctions. In a minority of T-cells, the TCRs consist of γ and δ delta chains. The TCR γ chain is generated by VJ recombination, and the TCR δ chain is generated by V(D)J recombination (Kenneth Murphy, Paul Travers, and Mark Walport, Janeway's Immunology 7th edition, Garland Science, 2007).

The DNA and RNA analyzed in the methods disclosed herein can correspond to sequences encoding heavy chain immunoglobulins (IgH) with constant regions (α, δ, ε, γ, or µ) or light chain immunoglobulins (IgK or IgL) with constant regions λ or κ. Each antibody has two identical light chains and two identical heavy chains. Each chain is composed of a constant (C) and a variable region. For the heavy chain, the variable region is composed of a variable (V), diversity (D), and joining (J) segments. Several distinct sequences coding for each type of these segments are present in the genome. A specific VDJ recombination event occurs during the development of a B-cell, marking that cell to generate a specific heavy chain. Diversity in the light chain is generated in a similar fashion except that there is no D region so there is only VJ recombination. Somatic mutation often occurs close to the site of the recombination, causing the addition or deletion of several nucleotides, further increasing the diversity of heavy and light chains generated by B-cells. The possible diversity of the antibodies generated by a B-cell is then the product of the different heavy and light chains. The variable regions of the heavy and light chains contribute to form the antigen recognition (or binding) region or site. Added to this diversity is a process of somatic hypermutation which can occur after a specific response is mounted against some epitope.

As mentioned above, primers may be selected to generate amplicons of subsets of recombined nucleic acids extracted from lymphocytes. Such subsets may be referred to herein as "somatically rearranged regions." Somatically rearranged regions may comprise nucleic acids from developing or from fully developed lymphocytes, where developing lymphocytes are cells in which rearrangement of immune genes has not been completed to form molecules having full V(D)J regions. Exemplary incomplete somatically rearranged regions include incomplete IgH molecules (such as, molecules containing only D-J regions), incomplete TCRδ molecules (such as, molecules containing only D-J regions), and inactive IgK (for example, comprising Kde-V regions).

Adequate sampling of the cells is an important aspect of interpreting the repertoire data, as described further below in the definitions of "clonotype" and "repertoire." For example, starting with 1,000 cells creates a minimum frequency that the assay is sensitive to regardless of how many sequencing reads are obtained. Therefore one aspect is the development of methods to quantitate the number of input immune receptor molecules. This has been implemented this for TCRβ and IgH sequences. In either case the same set of primers are used that are capable of amplifying all the different sequences. In order to obtain an absolute number of copies, a real time PCR with the multiplex of primers is performed along with a standard with a known number of immune receptor copies. This real time PCR measurement can be made from the amplification reaction that will subsequently be sequenced or can be done on a separate aliquot of the same sample. In the case of DNA, the absolute number of rearranged immune receptor molecules can be readily converted to number of cells (within 2 fold as some cells will have 2 rearranged copies of the specific immune receptor assessed and others will have one). In the case of cDNA the measured total number of rearranged molecules in the real time sample can be extrapolated to define the total number of these molecules used in another amplification reaction of the same sample. In addition, this method can be combined with a method to determine the total amount of RNA to define the number of rearranged immune receptor molecules in a unit amount (say 1 µg) of RNA assuming a specific efficiency of cDNA synthesis. If the total amount of cDNA is measured then the efficiency of cDNA synthesis need not be considered. If the number of cells is also known then the rearranged immune receptor copies per cell can be computed. If the number of cells is not known, one can estimate it from the total RNA as cells of specific type usually generate comparable amount of RNA. Therefore from the copies of rearranged immune receptor molecules per 1 µg one can estimate the number of these molecules per cell.

One disadvantage of doing a separate real time PCR from the reaction that would be processed for sequencing is that there might be inhibitory effects that are different in the real time PCR from the other reaction as different enzymes, input DNA, and other conditions may be utilized. Processing the products of the real time PCR for sequencing would ameliorate this problem. However low copy number using real time PCR can be due to either low number of copies or to inhibitory effects, or other suboptimal conditions in the reaction.

Another approach that can be utilized is to add a known amount of unique immune receptor rearranged molecules with a known sequence, i.e. known amounts of one or more internal standards, to the cDNA or genomic DNA from a sample of unknown quantity. By counting the relative number of molecules that are obtained for the known added sequence compared to the rest of the sequences of the same sample, one can estimate the number of rearranged immune receptor molecules in the initial cDNA sample. (Such techniques for molecular counting are well-known, e.g. Brenner et al, U.S. patent 7,537,897). Data from sequencing the added unique sequence can be used to distinguish the different possibilities if a real time PCR calibration is being used as well. Low copy number of rearranged immune receptor in the DNA (or cDNA) would create a high ratio between the number of molecules for the spiked sequence compared to the rest of the sample sequences. On the other hand, if the measured low copy number by real time PCR is due to inefficiency in the reaction, the ratio would not be high.

### Amplification of Nucleic Acid Populations

Amplicons of target populations of nucleic acids may be generated by a variety of amplification techniques. In one aspect of the invention, multiplex PCR is used to amplify members of a mixture of nucleic acids, particularly mixtures comprising recombined immune molecules such as T cell receptors, or portions thereof. Guidance for carrying out multiplex PCRs of such immune molecules is found in the following references: Morley, U.S. patent 5,296,351; Gorski, U.S. patent 5,837,447; Dau, U.S. patent 6,087,096; Von Dongen et al, U.S. patent publication 2006/0234234; European patent publication EP 1544308B1; and the like.

After amplification of DNA from the genome (or amplification of nucleic acid in the form of cDNA by reverse transcribing RNA), the individual nucleic acid molecules can be isolated, optionally re-amplified, and then sequenced individually. Exemplary amplification protocols may be found in van Dongen et al, Leukemia, 17: 2257-2317 (2003) or van Dongen et al, U.S. patent publication 2006/0234234. Briefly, an exemplary protocol is as follows: Reaction buffer: ABI Buffer II or ABI Gold Buffer (Life Technologies, San Diego, CA); 50 µL final reaction volume; 100 ng sample DNA; 10 pmol of each primer (subject to adjustments to balance amplification as described below); dNTPs at 200 µM final concentration; MgCl₂ at 1.5 mM final concentration (subject to optimization depending on target sequences and polymerase); Taq polymerase (1-2 U/tube); cycling conditions: preactivation 7 min at 95°C; annealing at 60°C; cycling times: 30s denaturation; 30s annealing; 30s extension. Polymerases that can be used for amplification in the methods of the invention are commercially available and include, for example, Taq polymerase, AccuPrime polymerase, or Pfu. The choice of polymerase to use can be based on whether fidelity or efficiency is preferred.

Real time PCR, picogreen staining, nanofluidic electrophoresis (e.g. LabChip) or UV absorption measurements can be used in an initial step to judge the functional amount of amplifiable material.

In one aspect, multiplex amplifications are carried out so that relative amounts of sequences in a starting population are substantially the same as those in the amplified population, or amplicon. That is, multiplex amplifications are carried out with minimal amplification bias among member sequences of a sample population. In one embodiment, such relative amounts are substantially the same if each relative amount in an amplicon is within five fold of its value in the starting sample. In another embodiment, such relative amounts are substantially the same if each relative amount in an amplicon is within two fold of its value in the starting sample. As discussed more fully below, amplification bias in PCR may be detected and corrected using conventional techniques so that a set of PCR primers may be selected for a predetermined repertoire that provide unbiased amplification of any sample.

In regard to many repertoires based on TCR or BCR sequences, a multiplex amplification optionally uses all the V segments. The reaction is optimized to attempt to get amplification that maintains the relative abundance of the sequences amplified by different V segment primers. Some of the primers are related, and hence many of the primers may "cross talk," amplifying templates that are not perfectly matched with it. The conditions are optimized so that each template can be amplified in a similar fashion irrespective of which primer amplified it. In other words if there are two templates, then after 1,000 fold amplification both templates can be amplified approximately 1,000 fold, and it does not matter that for one of the templates half of the amplified products carried a different primer because of the cross talk. In subsequent analysis of the sequencing data the primer sequence is eliminated from the analysis, and hence it does not matter what primer is used in the amplification as long as the templates are amplified equally.

In one embodiment, amplification bias may be avoided by carrying out a two-stage amplification (as described in Faham and Willis, cited above) wherein a small number of amplification cycles are implemented in a first, or primary, stage using primers having tails non-complementary with the target sequences. The tails include primer binding sites that are added to the ends of the sequences of the primary amplicon so that such sites are used in a second stage amplification using only a single forward primer and a single reverse primer, thereby eliminating a primary cause of amplification bias. In some embodiments, the primary PCR will have a small enough number of cycles (e.g. 5-10) to minimize the differential amplification by the different primers. The secondary amplification is done with one pair of primers, which minimizes differential amplification. In some embodiments, a small percent, e.g. one percent, of the primary PCR is taken directly to the secondary PCR. In some embodiments, a total of thirty-five cycles (equivalent to -28 cycles without the 100 fold dilution step) allocated between a first stage and a second stage are usually sufficient to show a robust amplification irrespective of whether the cycles are divided as follows: 1 cycle primary and 34 secondary; or 25 primary and 10 secondary.

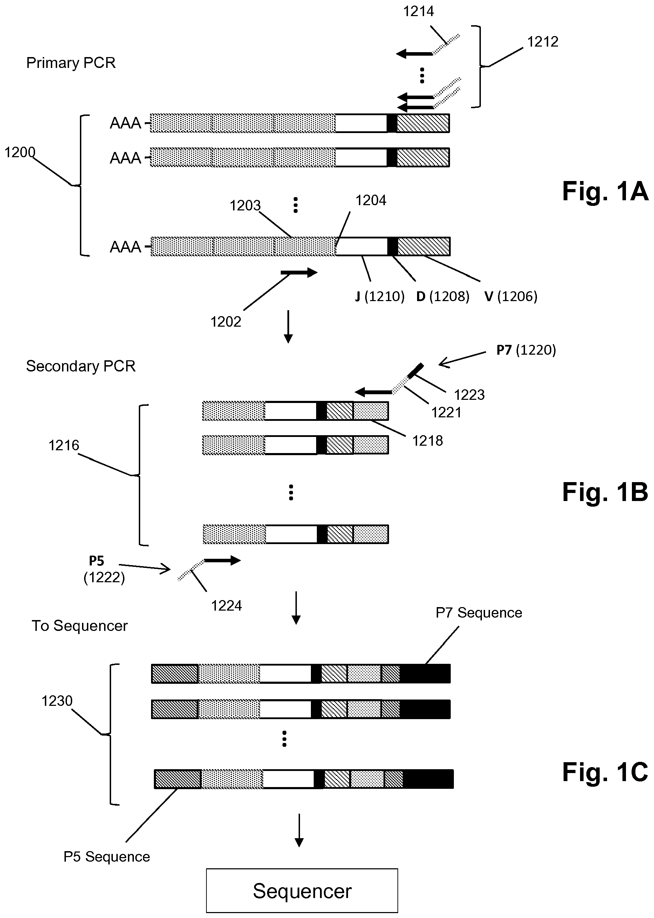

Briefly, the scheme of Faham and Willis (cited above) for amplifying IgH-encoding or TCRβ encoding nucleic acids (RNA) is illustrated in Figs. 1A-1C. Similar amplification schemes are readily for other immune receptor segments, e.g. Van Dongen et al, Leukemia, 17: 2257-2317 (2003), such as, incomplete IgH rearrangements, IgK, Kde, IgL, TCRγ, TCRδ, Bcl1-IgH, Bcl2-IgH, and the like. Nucleic acids (1200) are extracted from lymphocytes in a sample and combined in a PCR with a primer (1202) specific for C region (1203) and primers (1212) specific for the various V regions (1206) of the immunoglobulin or TCR genes. Primers (1212) each have an identical tail (1214) that provides a primer binding site for a second stage of amplification. As mentioned above, primer (1202) is positioned adjacent to junction (1204) between the C region (1203) and J region (1210). In the PCR, amplicon (1216) is generated that contains a portion of C-encoding region (1203), J-encoding region (1210), D-encoding region (1208), and a portion of V-encoding region (1206). Amplicon (1216) is further amplified in a second stage using primer P5 (1222) and primer P7 (1220), which each have tails (1225 and 1221/1223, respectively) designed for use in an Illumina DNA sequencer. Tail (1221/1223) of primer P7 (1220) optionally incorporates tag (1221) for labeling separate samples in the sequencing process. Second stage amplification produces amplicon (1230) which may be used in an Illumina DNA sequencer.

### Generating Sequence Reads

Any high-throughput technique for sequencing nucleic acids can be used in the method of the invention. Preferably, such technique has a capability of generating in a cost-effective manner a volume of sequence data from which at least 1000 clonotypes can be determined, and preferably, from which at least 10,000 to 1,000,000 clonotypes can be determined. DNA sequencing techniques include classic dideoxy sequencing reactions (Sanger method) using labeled terminators or primers and gel separation in slab or capillary, sequencing by synthesis using reversibly terminated labeled nucleotides, pyrosequencing, 454 sequencing, allele specific hybridization to a library of labeled oligonucleotide probes, sequencing by synthesis using allele specific hybridization to a library of labeled clones that is followed by ligation, real time monitoring of the incorporation of labeled nucleotides during a polymerization step, polony sequencing, and SOLiD sequencing. Sequencing of the separated molecules has more recently been demonstrated by sequential or single extension reactions using polymerases or ligases as well as by single or sequential differential hybridizations with libraries of probes. These reactions have been performed on many clonal sequences in parallel including demonstrations in current commercial applications of over 100 million sequences in parallel. These sequencing approaches can thus be used to study the repertoire of T-cell receptor (TCR) and/or B-cell receptor (BCR). In one aspect, high-throughput methods of sequencing are employed that comprise a step of spatially isolating individual molecules on a solid surface where they are sequenced in parallel. Such solid surfaces may include nonporous surfaces (such as in Solexa sequencing, e.g. Bentley et al, Nature,456: 53-59 (2008) or Complete Genomics sequencing, e.g. Drmanac et al, Science, 327: 78-81 (2010)), arrays of wells, which may include bead- or particle-bound templates (such as with 454, e.g. Margulies et al, Nature, 437: 376-380 (2005) or Ion Torrent sequencing, U.S. patent publication 2010/0137143 or 2010/0304982), micromachined membranes (such as with SMRT sequencing, e.g. Eid et al, Science, 323: 133-138 (2009)), or bead arrays (as with SOLiD sequencing or polony sequencing, e.g. Kim et al, Science, 316: 1481-1414 (2007)). In another aspect, such methods comprise amplifying the isolated molecules either before or after they are spatially isolated on a solid surface. Prior amplification may comprise emulsion-based amplification, such as emulsion PCR, or rolling circle amplification. Of particular interest is Solexa-based sequencing where individual template molecules are spatially isolated on a solid surface, after which they are amplified in parallel by bridge PCR to form separate clonal populations, or clusters, and then sequenced, as described in Bentley et al (cited above) and in manufacturer's instructions (e.g. TruSeq™ Sample Preparation Kit and Data Sheet, Illumina, Inc., San Diego, CA, 2010); and further in the following references: U.S. patents 6,090,592; 6,300,070; 7,115,400; and EP0972081B1. In one embodiment, individual molecules disposed and amplified on a solid surface form clusters in a density of at least 10⁵ clusters per cm²; or in a density of at least 5x10⁵ per cm²; or in a density of at least 10⁶ clusters per cm². In one embodiment, sequencing chemistries are employed having relatively high error rates. In such embodiments, the average quality scores produced by such chemistries are monotonically declining functions of sequence read lengths. In one embodiment, such decline corresponds to 0.5 percent of sequence reads have at least one error in positions 1-75; 1 percent of sequence reads have at least one error in positions 76-100; and 2 percent of sequence reads have at least one error in positions 101-125.

In one aspect, a sequence-based clonotype profile of an individual is obtained using the following steps: (a) obtaining a nucleic acid sample from T-cells and/or B-cells of the individual; (b) spatially isolating individual molecules derived from such nucleic acid sample, the individual molecules comprising at least one template generated from a nucleic acid in the sample, which template comprises a somatically rearranged region or a portion thereof, each individual molecule being capable of producing at least one sequence read; (c) sequencing said spatially isolated individual molecules; and (d) determining abundances of different sequences of the nucleic acid molecules from the nucleic acid sample to generate the clonotype profile. In one embodiment, each of the somatically rearranged regions comprise a V region and a J region. In another embodiment, the step of sequencing comprises bidirectionally sequencing each of the spatially isolated individual molecules to produce at least one forward sequence read and at least one reverse sequence read. Further to the latter embodiment, at least one of the forward sequence reads and at least one of the reverse sequence reads have an overlap region such that bases of such overlap region are determined by a reverse complementary relationship between such sequence reads. In still another embodiment, each of the somatically rearranged regions comprise a V region and a J region and the step of sequencing further includes determining a sequence of each of the individual nucleic acid molecules from one or more of its forward sequence reads and at least one reverse sequence read starting from a position in a J region and extending in the direction of its associated V region. In another embodiment, individual molecules comprise nucleic acids selected from the group consisting of complete IgH molecules, incomplete IgH molecules, complete IgK molecules, IgK inactive molecules, TCRβ molecules, TCRγ molecules, complete TCRδ molecules, and incomplete TCRδ molecules. In another embodiment, the step of sequencing comprises generating the sequence reads having monotonically decreasing quality scores. Further to the latter embodiment, monotonically decreasing quality scores are such that the sequence reads have error rates no better than the following: 0.2 percent of sequence reads contain at least one error in base positions 1 to 50, 0.2 to 1.0 percent of sequence reads contain at least one error in positions 51-75, 0.5 to 1.5 percent of sequence reads contain at least one error in positions 76-100. In another embodiment, the above method comprises the following steps: (a) obtaining a nucleic acid sample from T-cells and/or B-cells of the individual; (b) spatially isolating individual molecules derived from such nucleic acid sample, the individual molecules comprising nested sets of templates each generated from a nucleic acid in the sample and each containing a somatically rearranged region or a portion thereof, each nested set being capable of producing a plurality of sequence reads each extending in the same direction and each starting from a different position on the nucleic acid from which the nested set was generated; (c) sequencing said spatially isolated individual molecules; and (d) determining abundances of different sequences of the nucleic acid molecules from the nucleic acid sample to generate the clonotype profile. In one embodiment, the step of sequencing includes producing a plurality of sequence reads for each of the nested sets. In another embodiment, each of the somatically rearranged regions comprise a V region and a J region, and each of the plurality of sequence reads starts from a different position in the V region and extends in the direction of its associated J region.

In one aspect, for each sample from an individual, the sequencing technique used in the methods of the invention generates sequences of least 1000 clonotypes per run; in another aspect, such technique generates sequences of at least 10,000 clonotypes per run; in another aspect, such technique generates sequences of at least 100,000 clonotypes per run; in another aspect, such technique generates sequences of at least 500,000 clonotypes per run; and in another aspect, such technique generates sequences of at least 1,000,000 clonotypes per run. In still another aspect, such technique generates sequences of between 100,000 to 1,000,000 clonotypes per run per individual sample.

The sequencing technique used in the methods of the invention can generate about 30 bp, about 40 bp, about 50 bp, about 60 bp, about 70 bp, about 80 bp, about 90 bp, about 100 bp, about 110, about 120 bp per read, about 150 bp, about 200 bp, about 250 bp, about 300 bp, about 350 bp, about 400 bp, about 450 bp, about 500 bp, about 550 bp, or about 600 bp per read.

### Generating Clonotypes from Sequence Data

Constructing clonotypes from sequence read data is disclosed in Faham and Willis (cited above). Briefly, constructing clonotypes from sequence read data depends in part on the sequencing method used to generate such data, as the different methods have different expected read lengths and data quality. In one approach, a Solexa sequencer is employed to generate sequence read data for analysis. In one embodiment, a sample is obtained that provides at least 0.5-1.0x106 lymphocytes to produce at least 1 million template molecules, which after optional amplification may produce a corresponding one million or more clonal populations of template molecules (or clusters). For most high throughput sequencing approaches, including the Solexa approach, such over sampling at the cluster level is desirable so that each template sequence is determined with a large degree of redundancy to increase the accuracy of sequence determination. For Solexa-based implementations, preferably the sequence of each independent template is determined 10 times or more. For other sequencing approaches with different expected read lengths and data quality, different levels of redundancy may be used for comparable accuracy of sequence determination. Those of ordinary skill in the art recognize that the above parameters, e.g. sample size, redundancy, and the like, are design choices related to particular applications.

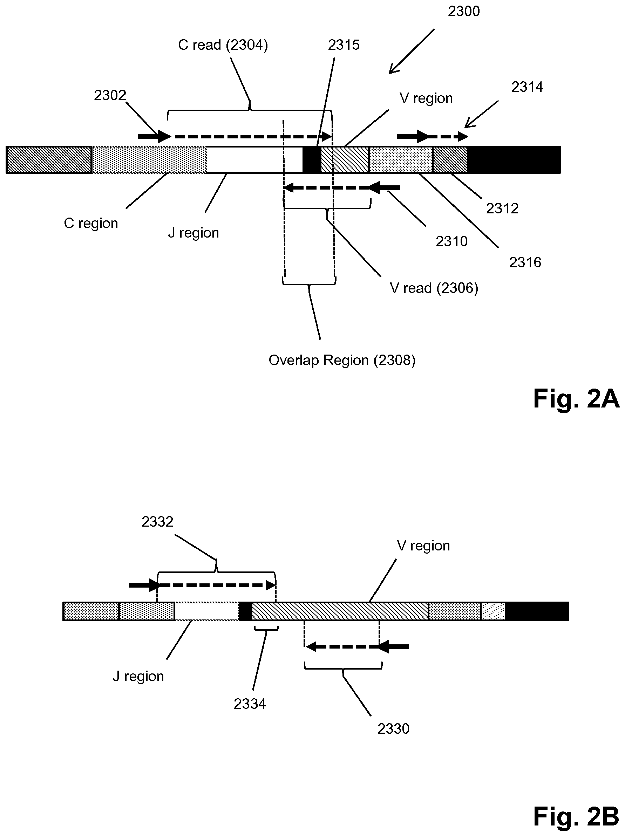

In one aspect, clonotypes of IgH chains or TCRβ chains (illustrated in Fig. 2A) are determined by at least one sequence read starting in its C region and extending in the direction of its associated V region (referred to herein as a "C read" (2304)) and at least one sequence read starting in its V region and extending in the direction of its associated J region (referred to herein as a "V read" (2306)). Such reads may or may not have an overlap region (2308) and such overlap may or may not encompass the NDN region (2315) as shown in Fig. 2A. Overlap region (2308) may be entirely in the J region, entirely in the NDN region, entirely in the V region, or it may encompass a J region-NDN region boundary or a V region-NDN region boundary, or both such boundaries (as illustrated in Fig. 2A). Typically, such sequence reads are generated by extending sequencing primers, e.g. (2302) and (2310) in Fig. 2A, with a polymerase in a sequencing-by-synthesis reaction, e.g. Metzger, Nature Reviews Genetics, 11: 31-46 (2010); Fuller et al, Nature Biotechnology, 27: 1013-1023 (2009). The binding sites for primers (2302) and (2310) are predetermined, so that they can provide a starting point or anchoring point for initial alignment and analysis of the sequence reads. In one embodiment, a C read is positioned so that it encompasses the D and/or NDN region of the IgH chain and includes a portion of the adjacent V region, e.g. as illustrated in Figs. 2A and 2B. In one aspect, the overlap of the V read and the C read in the V region is used to align the reads with one another. In other embodiments, such alignment of sequence reads is not necessary, so that a V read may only be long enough to identify the particular V region of a clonotype. This latter aspect is illustrated in Fig. 2B. Sequence read (2330) is used to identify a V region, with or without overlapping another sequence read, and another sequence read (2332) traverses the NDN region and is used to determine the sequence thereof. Portion (2334) of sequence read (2332) that extends into the V region is used to associate the sequence information of sequence read (2332) with that of sequence read (2330) to determine a clonotype. For some sequencing methods, such as base-by-base approaches like the Solexa sequencing method, sequencing run time and reagent costs are reduced by minimizing the number of sequencing cycles in an analysis. Optionally, as illustrated in Fig. 2A, amplicon (2300) is produced with sample tag (2312) to distinguish between clonotypes originating from different biological samples, e.g. different patients. Sample tag (2312) may be identified by annealing a primer to primer binding region (2316) and extending it (2314) to produce a sequence read across tag (2312), from which sample tag (2312) is decoded.

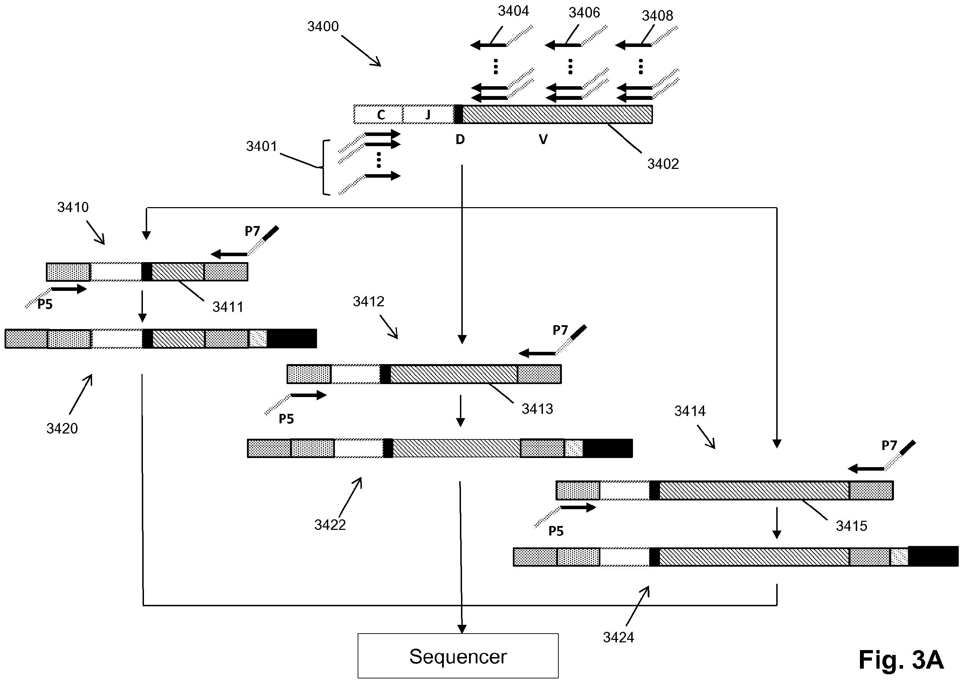

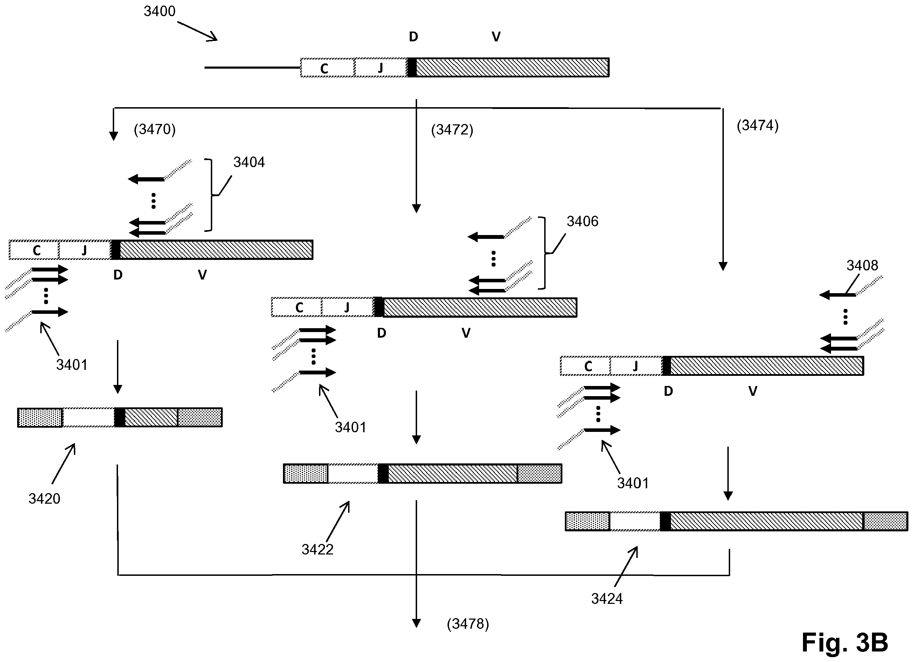

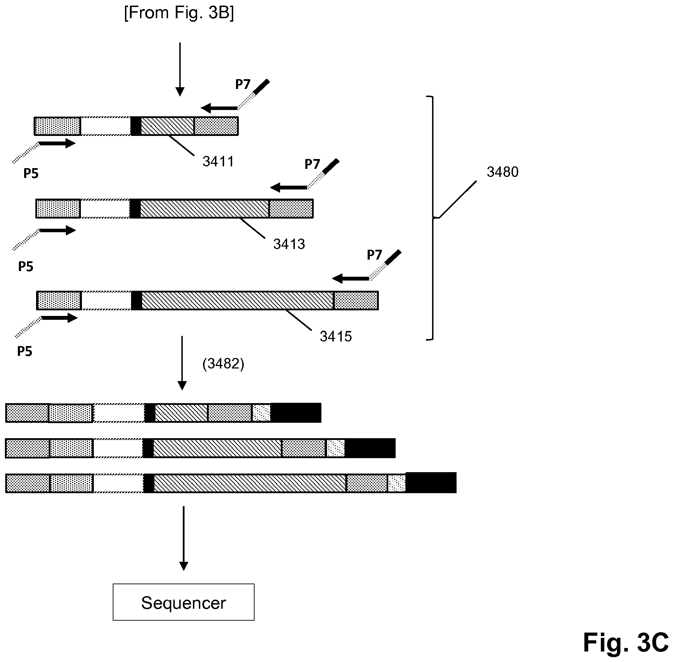

In one aspect, sequences of clonotypes may be determined by combining information from one or more sequence reads, for example, along the V(D)J regions of the selected chains. In another aspect, sequences of clonotypes are determined by combining information from a plurality of sequence reads. Such pluralities of sequence reads may include one or more sequence reads along a sense strand (i.e. "forward" sequence reads) and one or more sequence reads along its complementary strand (i.e. "reverse" sequence reads). When multiple sequence reads are generated along the same strand, separate templates are first generated by amplifying sample molecules with primers selected for the different positions of the sequence reads. This concept is illustrated in Fig. 3A where primers (3404, 3406 and 3408) are employed to generate amplicons (3410, 3412, and 3414, respectively) in a single reaction. Such amplifications may be carried out in the same reaction or in separate reactions. In one aspect, whenever PCR is employed, separate amplification reactions are used for generating the separate templates which, in turn, are combined and used to generate multiple sequence reads along the same strand. This latter approach is preferable for avoiding the need to balance primer concentrations (and/or other reaction parameters) to ensure equal amplification of the multiple templates (sometimes referred to herein as "balanced amplification" or "unbias amplification"). The generation of templates in separate reactions is illustrated in Figs. 3B-3C. There a sample containing IgH (3400) is divided into three portions (3470, 3472, and 3474) which are added to separate PCRs using J region primers (3401) and V region primers (3404, 3406, and 3408, respectively) to produce amplicons (3420, 3422 and 3424, respectively). The latter amplicons are then combined (3478) in secondary PCR (3480) using P5 and P7 primers to prepare the templates (3482) for bridge PCR and sequencing on an Illumina GA sequencer, or like instrument.

Sequence reads may have a wide variety of lengths, depending in part on the sequencing technique being employed. For example, for some techniques, several trade-offs may arise in its implementation, for example, (i) the number and lengths of sequence reads per template and (ii) the cost and duration of a sequencing operation. In one embodiment, sequence reads are in the range of from 20 to 200 nucleotides; in another embodiment, sequence reads are in a range of from 30 to 200 nucleotides; in still another embodiment, sequence reads are in the range of from 30 to 120 nucleotides. In one embodiment, 1 to 4 sequence reads are generated for determining the sequence of each clonotype; in another embodiment, 2 to 4 sequence reads are generated for determining the sequence of each clonotype; and in another embodiment, 2 to 3 sequence reads are generated for determining the sequence of each clonotype. In some embodiments, a plurality of sequence reads are used to generate each clonotype; in some embodiments, the plurality of sequence reads used to generate a clonotype is at least 10; in other embodiments, the plurality of sequence reads used to generate a clonotype is at least 20. In some embodiments, the plurality of sequence reads used to generate a clonotype is a number required for determining that different clonotypes are different with a confidence level of at least 99 percent; in other embodiments, the plurality of sequence reads used to generate a clonotype is a number required for determining that different clonotypes are different with a confidence level of at least 99.9 percent. As noted below, clonotypes may be generated from sequence reads by a step of combining, or as sometimes referred to herein, a step of coalescing, sequence reads in accordance with a procedure (as exemplified in the disclosure below) that takes into account sequencing and/or amplification error rates, sequence read frequencies, sequence differences, and the like. In the foregoing embodiments, the numbers given are exclusive of sequence reads used to identify samples from different individuals. The lengths of the various sequence reads used in the embodiments described below may also vary based on the information that is sought to be captured by the read; for example, the starting location and length of a sequence read may be designed to provide the length of an NDN region as well as its nucleotide sequence; thus, sequence reads spanning the entire NDN region are selected. In other aspects, one or more sequence reads that in combination (but not separately) encompass a D and /or NDN region are sufficient.

In another aspect, sequences of clonotypes are determined in part by aligning sequence reads to one or more V region reference sequences and one or more J region reference sequences, and in part by base determination without alignment to reference sequences, such as in the highly variable NDN region. A variety of alignment algorithms may be applied to the sequence reads and reference sequences. For example, guidance for selecting alignment methods is available in Batzoglou, Briefings in Bioinformatics, 6: 6-22 (2005). In one aspect, whenever V reads or C reads (as mentioned above) are aligned to V and J region reference sequences, a tree search algorithm is employed, e.g. as described generally in Gusfield (cited above) and Cormen et al, Introduction to Algorithms, Third Edition (The MIT Press, 2009).

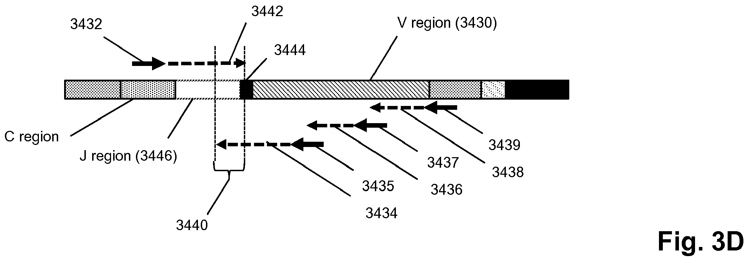

The construction of IgH clonotypes from sequence reads is characterized by at least two factors: i) the presence of somatic mutations which makes alignment more difficult, and ii) the NDN region is larger so that it is often not possible to map a portion of the V segment to the C read. In one aspect, this problem is overcome by using a plurality of primer sets for generating V reads, which are located at different locations along the V region, preferably so that the primer binding sites are nonoverlapping and spaced apart, and with at least one primer binding site adjacent to the NDN region, e.g. in one embodiment from 5 to 50 bases from the V-NDN junction, or in another embodiment from 10 to 50 bases from the V-NDN junction. The redundancy of a plurality of primer sets minimizes the risk of failing to detect a clonotype due to a failure of one or two primers having binding sites affected by somatic mutations. In addition, the presence of at least one primer binding site adjacent to the NDN region makes it more likely that a V read will overlap with the C read and hence effectively extend the length of the C read. This allows for the generation of a continuous sequence that spans all sizes of NDN regions and that can also map substantially the entire V and J regions on both sides of the NDN region. Embodiments for carrying out such a scheme are illustrated in Figs. 3A and 3D. In Fig. 3A, a sample comprising IgH chains (3400) are sequenced by generating a plurality amplicons for each chain by amplifying the chains with a single set of J region primers (3401) and a plurality (three shown) of sets of V region (3402) primers (3404, 3406, 3408) to produce a plurality of nested amplicons (e.g., 3410, 3412, 3416) all comprising the same NDN region and having different lengths encompassing successively larger portions (3411, 3413, 3415) of V region (3402). Members of a nested set may be grouped together after sequencing by noting the identify (or substantial identity) of their respective NDN, J and/or C regions, thereby allowing reconstruction of a longer V(D)J segment than would be the case otherwise for a sequencing platform with limited read length and/or sequence quality. In one embodiment, the plurality of primer sets may be a number in the range of from 2 to 5. In another embodiment the plurality is 2-3; and still another embodiment the plurality is 3. The concentrations and positions of the primers in a plurality may vary widely. Concentrations of the V region primers may or may not be the same. In one embodiment, the primer closest to the NDN region has a higher concentration than the other primers of the plurality, e.g. to insure that amplicons containing the NDN region are represented in the resulting amplicon. In a particular embodiment where a plurality of three primers is employed, a concentration ratio of 60:20:20 is used. One or more primers (e.g. 3435 and 3437 in Fig. 3D) adjacent to the NDN region (3444) may be used to generate one or more sequence reads (e.g. 3434 and 3436) that overlap the sequence read (3442) generated by J region primer (3432), thereby improving the quality of base calls in overlap region (3440). Sequence reads from the plurality of primers may or may not overlap the adjacent downstream primer binding site and/or adjacent downstream sequence read. In one embodiment, sequence reads proximal to the NDN region (e.g. 3436 and 3438) may be used to identify the particular V region associated with the clonotype. Such a plurality of primers reduces the likelihood of incomplete or failed amplification in case one of the primer binding sites is hypermutated during immunoglobulin development. It also increases the likelihood that diversity introduced by hypermutation of the V region will be capture in a clonotype sequence. A secondary PCR may be performed to prepare the nested amplicons for sequencing, e.g. by amplifying with the P5 (3401) and P7 (3404, 3406, 3408) primers as illustrated to produce amplicons (3420, 3422, and 3424), which may be distributed as single molecules on a solid surface, where they are further amplified by bridge PCR, or like technique.

Somatic Hypermutations. In one embodiment, IgH-based clonotypes that have undergone somatic hypermutation are determined as follows. A somatic mutation is defined as a sequenced base that is different from the corresponding base of a reference sequence (of the relevant segment, usually V, J or C) and that is present in a statistically significant number of reads. In one embodiment, C reads may be used to find somatic mutations with respect to the mapped J segment and likewise V reads for the V segment. Only pieces of the C and V reads are used that are either directly mapped to J or V segments or that are inside the clonotype extension up to the NDN boundary. In this way, the NDN region is avoided and the same 'sequence information' is not used for mutation finding that was previously used for clonotype determination (to avoid erroneously classifying as mutations nucleotides that are really just different recombined NDN regions). For each segment type, the mapped segment (major allele) is used as a scaffold and all reads are considered which have mapped to this allele during the read mapping phase. Each position of the reference sequences where at least one read has mapped is analyzed for somatic mutations. In one embodiment, the criteria for accepting a non-reference base as a valid mutation include the following: 1) at least N reads with the given mutation base, 2) at least a given fraction N/M reads (where M is the total number of mapped reads at this base position) and 3) a statistical cut based on the binomial distribution, the average Q score of the N reads at the mutation base as well as the number (M-N) of reads with a non-mutation base. Preferably, the above parameters are selected so that the false discovery rate of mutations per clonotype is less than 1 in 1000, and more preferably, less than 1 in 10000.

It is expected that PCR error is concentrated in some bases that were mutated in the early cycles of PCR. Sequencing error is expected to be distributed in many bases even though it is totally random as the error is likely to have some systematic biases. It is assumed that some bases will have sequencing error at a higher rate, say 5% (5 fold the average). Given these assumptions, sequencing error becomes the dominant type of error. Distinguishing PCR errors from the occurrence of highly related clonotypes will play a role in analysis. Given the biological significance to determining that there are two or more highly related clonotypes, a conservative approach to making such calls is taken. The detection of enough of the minor clonotypes so as to be sure with high confidence (say 99.9%) that there are more than one clonotype is considered. For example of clonotypes that are present at 100 copies/1,000,000, the minor variant is detected 14 or more times for it to be designated as an independent clonotype. Similarly, for clonotypes present at 1,000 copies/1,000,000 the minor variant can be detected 74 or more times to be designated as an independent clonotype. This algorithm can be enhanced by using the base quality score that is obtained with each sequenced base. If the relationship between quality score and error rate is validated above, then instead of employing the conservative 5% error rate for all bases, the quality score can be used to decide the number of reads that need to be present to call an independent clonotype. The median quality score of the specific base in all the reads can be used, or more rigorously, the likelihood of being an error can be computed given the quality score of the specific base in each read, and then the probabilities can be combined (assuming independence) to estimate the likely number of sequencing error for that base. As a result, there are different thresholds of rejecting the sequencing error hypothesis for different bases with different quality scores. For example for a clonotype present at 1,000 copies/1,000,000 the minor variant is designated independent when it is detected 22 and 74 times if the probability of error were 0.01 and 0.05, respectively.

In the presence of sequencing errors, each genuine clonotype is surrounded by a 'cloud' of reads with varying numbers of errors with respect to the its sequence. The "cloud" of sequencing errors drops off in density as the distance increases from the clonotype in sequence space. A variety of algorithms are available for converting sequence reads into clonotypes. In one aspect, coalescing of sequence reads (that is, merging candidate clonotypes determined to have one or more sequencing errors) depends on at least three factors: the number of sequences obtained for each of the clonotypes being compared; the number of bases at which they differ; and the sequencing quality score at the positions at which they are discordant. A likelihood ratio may be constructed and assessed that is based on the expected error rates and binomial distribution of errors. For example, two clonotypes, one with 150 reads and the other with 2 reads with one difference between them in an area of poor sequencing quality will likely be coalesced as they are likely to be generated by sequencing error. On the other hand two clonotypes, one with 100 reads and the other with 50 reads with two differences between them are not coalesced as they are considered to be unlikely to be generated by sequencing error. In one embodiment, the algorithm described below may be used for determining clonotypes from sequence reads. In one aspect, sequence reads are first converted into candidate clonotypes. Such a conversion depends on the sequencing platform employed. For platforms that generate high Q score long sequence reads, the sequence read or a portion thereof may be taken directly as a candidate clonotype. For platforms that generate lower Q score shorter sequence reads, some alignment and assembly steps may be required for converting a set of related sequence reads into a candidate clonotype. For example, for Solexa-based platforms, in some embodiments, candidate clonotypes are generated from collections of paired reads from multiple clusters, e.g. 10 or more, as mentioned above

The cloud of sequence reads surrounding each candidate clonotype can be modeled using the binomial distribution and a simple model for the probability of a single base error. This latter error model can be inferred from mapping V and J segments or from the clonotype finding algorithm itself, via self-consistency and convergence. A model is constructed for the probability of a given 'cloud' sequence Y with read count C2 and E errors (with respect to sequence X) being part of a true clonotype sequence X with perfect read count C1 under the null model that X is the only true clonotype in this region of sequence space. A decision is made whether or not to coalesce sequence Y into the clonotype X according the parameters C1, C2, and E. For any given C1 and E a max value C2 is pre-calculated for deciding to coalesce the sequence Y. The max values for C2 are chosen so that the probability of failing to coalesce Y under the null hypothesis that Y is part of clonotype X is less than some value P after integrating over all possible sequences Y with error E in the neighborhood of sequence X. The value P is controls the behavior of the algorithm and makes the coalescing more or less permissive.

If a sequence Y is not coalesced into clonotype X because its read count is above the threshold C2 for coalescing into clonotype X then it becomes a candidate for seeding separate clonotypes. An algorithm implementing such principles makes sure that any other sequences Y2, Y3, etc. which are 'nearer' to this sequence Y (that had been deemed independent of X) are not aggregated into X. This concept of 'nearness' includes both error counts with respect to Y and X and the absolute read count of X and Y, i.e. it is modeled in the same fashion as the above model for the cloud of error sequences around clonotype X. In this way 'cloud' sequences can be properly attributed to their correct clonotype if they happen to be 'near' more than one clonotype.

In one embodiment, an algorithm proceeds in a top down fashion by starting with the sequence X with the highest read count. This sequence seeds the first clonotype. Neighboring sequences are either coalesced into this clonotype if their counts are below the precalculated thresholds (see above), or left alone if they are above the threshold or 'closer' to another sequence that was not coalesced. After searching all neighboring sequences within a maximum error count, the process of coalescing reads into clonotype X is finished. Its reads and all reads that have been coalesced into it are accounted for and removed from the list of reads available for making other clonotypes. The next sequence is then moved on to with the highest read count. Neighboring reads are coalesced into this clonotype as above and this process is continued until there are no more sequences with read counts above a given threshold, e.g. until all sequences with more than 1 count have been used as seeds for clonotypes.

As mentioned above, in another embodiment of the above algorithm, a further test may be added for determining whether to coalesce a candidate sequence Y into an existing clonotype X, which takes into account quality score of the relevant sequence reads. The average quality score(s) are determined for sequence(s) Y (averaged across all reads with sequence Y) were sequences Y and X differ. If the average score is above a predetermined value then it is more likely that the difference indicates a truly different clonotype that should not be coalesced and if the average score is below such predetermined value then it is more likely that sequence Y is caused by sequencing errors and therefore should be coalesced into X.

### Related Clonotypes

Frequently lymphocytes produce related clonotypes. That is, multiple lymphocytes may exist or develop that produce clonotypes whose sequences are similar. This may be due to a variety of mechanism, such as hypermutation in the case of IgH molecules. As another example, in cancers, such as lymphoid neoplasms, a single lymphocyte progenitor may give rise to many related lymphocyte progeny, each possessing and/or expressing a slightly different TCR or BCR, and therefore a different clonotype, due to cancer-related somatic mutation(s), such as base substitutions, aberrant rearrangements, or the like. A set of such related clonotypes is referred to herein as a "clan." In some case, clonotypes of a clan may arise from the mutation of another clan member. Such an "offspring" clonotype may be referred to as a phylogenic clonotype. Clonotypes within a clan may be identified by one or more measures of relatedness to a parent clonotype, or to each other. In one embodiment, clonotypes may be grouped into the same clan by percent homology, as described more fully below. In another embodiment, clonotypes may be assigned to a clan by common usage of V regions, J regions, and/or NDN regions. For example, a clan may be defined by clonotypes having common J and ND regions but different V regions; or it may be defined by clonotypes having the same V and J regions (including identical base substitutions mutations) but with different NDN regions; or it may be defined by a clonotype that has undergone one or more insertions and/or deletions of from 1-10 bases, or from 1-5 bases, or from 1-3 bases, to generate clan members. In another embodiment, members of a clan are determined as follows.

Clonotypes are assigned to the same clan if they satisfy the following criteria: i) they are mapped to the same V and J reference segments, with the mappings occurring at the same relative positions in the clonotype sequence, and ii) their NDN regions are substantially identical. "Substantial" in reference to clan membership means that some small differences in the NDN region are allowed because somatic mutations may have occurred in this region. Preferably, in one embodiment, to avoid falsely calling a mutation in the NDN region, whether a base substitution is accepted as a cancer-related mutation depends directly on the size of the NDN region of the clan. For example, a method may accept a clonotype as a clan member if it has a one-base difference from clan NDN sequence(s) as a cancer-related mutation if the length of the clan NDN sequence(s) is m nucleotides or greater, e.g. 9 nucleotides or greater, otherwise it is not accepted, or if it has a two-base difference from clan NDN sequence(s) as cancer-related mutations if the length of the clan NDN sequence(s) is n nucleotides or greater, e.g. 20 nucleotides or greater, otherwise it is not accepted, In another embodiment, members of a clan are determined using the following criteria: (a) V read maps to the same V region, (b) C read maps to the same J region, (c) NDN region substantially identical (as described above), and (d) position of NDN region between V-NDN boundary and J-NDN boundary is the same (or equivalently, the number of downstream base additions to D and the number of upstream base additions to D are the same). Clonotypes of a single sample may be grouped into clans and clans from successive samples acquired at different times may be compared with one another. In particular, in one aspect, clans containing clonotypes correlated with a disease, such as a lymphoid neoplasm, are identified from clonotypes of each sample and compared with that of the immediately previous sample to determine disease status, such as, continued remission, incipient relapse, evidence of further clonal evolution, or the like. As used herein, "size" in reference to a clan means the number of clonotypes in the clan.

As mentioned above, in one aspect, methods disclosed herein monitor a level of a clan of clonotypes rather than an individual clonotype. This is because of the phenomena of clonal evolution, e.g. Campbell et al, Proc. Natl. Acad. Sci., 105: 13081-13086 (2008); Gerlinger et al, Br. J. Cancer, 103: 1139-1143 (2010). The sequence of a clone that is present in the diagnostic sample may not remain exactly the same as the one in a later sample, such as one taken upon a relapse of disease. Therefore if one is following the exact clonotype sequence that matches the diagnostic sample sequence, the detection of a relapse might fail. Such evolved clone are readily detected and identified by sequencing. For example many of the evolved clones emerge by V region replacement (called VH replacement). These types of evolved clones are missed by real time PCR techniques since the primers target the wrong V segment. However given that the D-J junction stays intact in the evolved clone, it can be detected and identified using the sequencing of individual spatially isolated molecules. Furthermore, the presence of these related clonotypes at appreciable frequency in the diagnostic sample increases the likelihood of the relevance of the clonotype. Similarly the development of somatic hypermutations in the immune receptor sequence may interfere with the real time PCR probe detection, but appropriate algorithms applied to the sequencing readout (as disclosed above) can still recognize a clonotype as an evolving clonotype. For example, somatic hypermutations in the V or J segments can be recognized. This is done by mapping the clonotypes to the closest germ line V and J sequences. Differences from the germ line sequences can be attributed to somatic hypermutations. Therefore clonotypes that evolve through somatic hypermutations in the V or J segments can be readily detected and identified. Somatic hypermutations in the NDN region can be predicted. When the remaining D segment is long enough to be recognized and mapped, any somatic mutation in it can be readily recognized. Somatic hypermutations in the N+P bases (or in D segment that is not mappable) cannot be recognized for certain as these sequences can be modified in newly recombined cells which may not be progeny of the cancerous clonotype. However algorithms are readily constructed to identify base changes that have a high likelihood of being due to somatic mutation. For example a clonotype with the same V and J segments and 1 base difference in the NDN region from the original clone(s) has a high likelihood of being the result of somatic recombination. This likelihood can be increased if there are other somatic hypermutations in the V and J segments because this identifies this specific clonotype as one that has been the subject of somatic hypermutation. Therefore the likelihood of a clonotype being the result of somatic hypermutation from an original clonotype can be computed using several parameters: the number of differences in the NDN region, the length of NDN region, as well as the presence of other somatic hypermutations in the V and/or J segments.

The clonal evolution data can be informative. For example if the major clone is an evolved clone (one that was absent previously, and therefore, previously unrecorded) then this is an indication of that tumor has acquired new genetic changes with potential selective advantages. This is not to say that the specific changes in the immune cell receptor are the cause of the selective advantage but rather that they may represent a marker for it. Tumors whose clonotypes have evolved can potentially be associated with differential prognosis. In one aspect, a clonotype or clonotypes being used as a patient-specific biomarker of a disease, such as a lymphoid neoplasm, for example, a leukemia, includes previously unrecorded clonotypes that are somatic mutants of the clonotype or clonotypes being monitored. In another aspect, whenever any previously unrecorded clonotype is at least ninety percent homologous to an existing clonotype or group of clonotypes serving as patient-specific biomarkers, then such homologous clonotype is included with or in the group of clonotypes being monitored going forward. That is, if one or more patient-specific clonotypes are identified in a lymphoid neoplasm and used to periodically monitor the disease (for example, by making measurement on less invasively acquired blood samples) and if in the course of one such measurement a new (previously unrecorded) clonotype is detected that is a somatic mutation of a clonotype of the current set, then it is added to the set of patient-specific clonotypes that are monitored for subsequent measurements. In one embodiment, if such previously unrecorded clonotype is at least ninety percent homologous with a member of the current set, then it is added to the patient-specific set of clonotype biomarkers for the next test carried out on the patient; that is, the such previously unrecorded clonotype is included in the clan of the member of the current set of clonotypes from which it was derived (based on the above analysis of the clonotype data). In another embodiment, such inclusion is carried out if the previously unrecorded clonotype is at least ninety-five percent homologous with a member of the current set. In another embodiment, such inclusion is carried out if the previously unrecorded clonotype is at least ninety-eight percent homologous with a member of the current set.

It is also possible that a cell evolves through a process that replaces the NDN region but preserves the V and J segment along with their accumulated mutations. Such cells can be identified as previously unrecorded cancer clonotypes by the identification of the common V and J segment provided they contain a sufficient numer of mutations to render the chance of these mutations being independently derived small. A further constraint may be that the NDN region is of similar size to the preoviously sequenced clone.

### Definitions

Unless otherwise specifically defined herein, terms and symbols of nucleic acid chemistry, biochemistry, genetics, and molecular biology used herein follow those of standard treatises and texts in the field, e.g. Kornberg and Baker, DNA Replication, Second Edition (W.H. Freeman, New York, 1992); Lehninger, Biochemistry, Second Edition (Worth Publishers, New York, 1975); Strachan and Read, Human Molecular Genetics, Second Edition (Wiley-Liss, New York, 1999); Abbas et al, Cellular and Molecular Immunology, 6th edition (Saunders, 2007).
"Aligning" means a method of comparing a test sequence, such as a sequence read, to one or more reference sequences to determine which reference sequence or which portion of a reference sequence is closest based on some sequence distance measure. An exemplary method of aligning nucleotide sequences is the Smith Waterman algorithm. Distance measures may include Hamming distance, Levenshtein distance, or the like. Distance measures may include a component related to the quality values of nucleotides of the sequences being compared.
"Amplicon" means the product of a polynucleotide amplification reaction; that is, a clonal population of polynucleotides, which may be single stranded or double stranded, which are replicated from one or more starting sequences. The one or more starting sequences may be one or more copies of the same sequence, or they may be a mixture of different sequences. Preferably, amplicons are formed by the amplification of a single starting sequence. Amplicons may be produced by a variety of amplification reactions whose products comprise replicates of the one or more starting, or target, nucleic acids. In one aspect, amplification reactions producing amplicons are "template-driven" in that base pairing of reactants, either nucleotides or oligonucleotides, have complements in a template polynucleotide that are required for the creation of reaction products. In one aspect, template-driven reactions are primer extensions with a nucleic acid polymerase or oligonucleotide ligations with a nucleic acid ligase. Such reactions include, but are not limited to, polymerase chain reactions (PCRs), linear polymerase reactions, nucleic acid sequence-based amplification (NASBAs), rolling circle amplifications, and the like, disclosed in the following references : Mullis et al, U.S. patents 4,683,195; 4,965,188; 4,683,202; 4,800,159 (PCR); Gelfand et al, U.S. patent 5,210,015 (real-time PCR with "taqman" probes); Wittwer et al, U.S. patent 6,174,670; Kacian et al, U.S. patent 5,399,491 ("NASBA"); Lizardi, U.S. patent 5,854,033; Aono et al, Japanese patent publ. JP 4-262799 (rolling circle amplification); and the like. In one aspect, amplicons are produced by PCRs. An amplification reaction may be a "real-time" amplification if a detection chemistry is available that permits a reaction product to be measured as the amplification reaction progresses, e.g. "real-time PCR" described below, or "real-time NASBA" as described in Leone et al, Nucleic Acids Research, 26: 2150-2155 (1998), and like references. As used herein, the term "amplifying" means performing an amplification reaction. A "reaction mixture" means a solution containing all the necessary reactants for performing a reaction, which may include, but not be limited to, buffering agents to maintain pH at a selected level during a reaction, salts, co-factors, scavengers, and the like.
"Clonality" as used herein means a measure of the degree to which the distribution of clonotype abundances among clonotypes of a repertoire is skewed to a single or a few clonotypes. Roughly, clonality is an inverse measure of clonotype diversity. Many measures or statistics are available from ecology describing species-abundance relationships that may be used for clonality measures, e.g. Chapters 17 & 18, in Pielou, An Introduction to Mathematical Ecology, (Wiley-Interscience, 1969). In one aspect, a clonality measure is a function of a clonotype profile (that is, the number of distinct clonotypes detected and their abundances), so that after a clonotype profile is measured, clonality may be computed from it to give a single number. One clonality measure is Simpson's measure, which is simply the probability that two randomly drawn clonotypes will be the same. Other clonality measures include information-based measures and McIntosh's diversity index, disclosed in Pielou (cited above).
"Clonotype" means a recombined nucleotide sequence of a lymphocyte which encodes an immune receptor or a portion thereof. More particularly, clonotype means a recombined nucleotide sequence of a T cell or B cell which encodes a T cell receptor (TCR) or B cell receptor (BCR), or a portion thereof. In various embodiments, clonotypes may encode all or a portion of a VDJ rearrangement of IgH, a DJ rearrangement of IgH, a VJ rearrangement of IgK, a VJ rearrangement of IgL, a VDJ rearrangement of TCR β, a DJ rearrangement of TCR β, a VJ rearrangement of TCR α, a VJ rearrangement of TCR γ, a VDJ rearrangement of TCR δ, a VD rearrangement of TCR δ, a Kde-V rearrangement, or the like. Clonotypes may also encode translocation breakpoint regions involving immune receptor genes, such as Bcl1-IgH or Bcl1-IgH. In one aspect, clonotypes have sequences that are sufficiently long to represent or reflect the diversity of the immune molecules that they are derived from; consequently, clonotypes may vary widely in length. In some embodiments, clonotypes have lengths in the range of from 25 to 400 nucleotides; in other embodiments, clonotypes have lengths in the range of from 25 to 200 nucleotides.
"Clonotype profile" means a listing of distinct clonotypes and their relative abundances that are derived from a population of lymphocytes. Typically, the population of lymphocytes are obtained from a tissue sample. The term "clonotype profile" is related to, but more general than, the immunology concept of immune "repertoire" as described in references, such as the following: Arstila et al, Science, 286: 958-961 (1999); Yassai et al, Immunogenetics, 61: 493-502 (2009); Kedzierska et al, Mol. Immunol., 45(3): 607-618 (2008); and the like. The term "clonotype profile" includes a wide variety of lists and abundances of rearranged immune receptor-encoding nucleic acids, which may be derived from selected subsets of lymphocytes (e.g. tissue-infiltrating lymphocytes, immunophenotypic subsets, or the like), or which may encode portions of immune receptors that have reduced diversity as compared to full immune receptors. In some embodiments, clonotype profiles may comprise at least 10³ distinct clonotypes; in other embodiments, clonotype profiles may comprise at least 10⁴ distinct clonotypes; in other embodiments, clonotype profiles may comprise at least 10⁵ distinct clonotypes; in other embodiments, clonotype profiles may comprise at least 10⁶ distinct clonotypes. In such embodiments, such clonotype profiles may further comprise abundances or relative frequencies of each of the distinct clonotypes. In one aspect, a clonotype profile is a set of distinct recombined nucleotide sequences (with their abundances) that encode T cell receptors (TCRs) or B cell receptors (BCRs), or fragments thereof, respectively, in a population of lymphocytes of an individual, wherein the nucleotide sequences of the set have a one-to-one correspondence with distinct lymphocytes or their clonal subpopulations for substantially all of the lymphocytes of the population. In one aspect, nucleic acid segments defining clonotypes are selected so that their diversity (i.e. the number of distinct nucleic acid sequences in the set) is large enough so that substantially every T cell or B cell or clone thereof in an individual carries a unique nucleic acid sequence of such repertoire. That is, preferably each different clone of a sample has different clonotype. The population of lymphocytes corresponding to a repertoire may be circulating B cells, or may be circulating T cells, or may be subpopulations of either of the foregoing populations, including but not limited to, CD4+ T cells, or CD8+ T cells, or other subpopulations defined by cell surface markers, or the like. Such subpopulations may be acquired by taking samples from particular tissues, e.g. bone marrow, or lymph nodes, or the like, or by sorting or enriching cells from a sample (such as peripheral blood) based on one or more cell surface markers, size, morphology, or the like. In still other aspects, the population of lymphocytes corresponding to a repertoire may be derived from disease tissues, such as a tumor tissue, an infected tissue, or the like. In one embodiment, a clonotype profile comprising human TCR β chains or fragments thereof comprises a number of distinct nucleotide sequences in the range of from 0.1 x 10⁶ to 1.8 x 10⁶, or in the range of from 0.5 x 10⁶ to 1.5 x 10⁶, or in the range of from 0.8 x 10⁶ to 1.2 x 10⁶. In another embodiment, a clonotype profile comprising human IgH chains or fragments thereof comprises a number of distinct nucleotide sequences in the range of from 0.1 x 10⁶ to 1.8 x 10⁶, or in the range of from 0.5 x 10⁶ to 1.5 x 10⁶, or in the range of from 0.8 x 10⁶ to 1.2 x 10⁶. In a particular embodiment, a clonotype profile comprises a set of nucleotide sequences encoding substantially all segments of the V(D)J region of an IgH chain. In one aspect, "substantially all" as used herein means every segment having a relative abundance of .001 percent or higher; or in another aspect, "substantially all" as used herein means every segment having a relative abundance of .0001 percent or higher. In another particular embodiment, a clonotype profile comprises a set of nucleotide sequences that encodes substantially all segments of the V(D)J region of a TCR β chain. In another embodiment, a clonotype profile comprises a set of nucleotide sequences having lengths in the range of from 25-200 nucleotides and including segments of the V, D, and J regions of a TCR β chain. In another embodiment, a clonotype profile comprises a set of nucleotide sequences having lengths in the range of from 25-200 nucleotides and including segments of the V, D, and J regions of an IgH chain. In another embodiment, a clonotype profile comprises a number of distinct nucleotide sequences that is substantially equivalent to the number of lymphocytes expressing a distinct IgH chain. In another embodiment, a clonotype profile comprises a number of distinct nucleotide sequences that is substantially equivalent to the number of lymphocytes expressing a distinct TCR β chain. In still another embodiment, "substantially equivalent" means that with ninety-nine percent probability a clonotype profile will include a nucleotide sequence encoding an IgH or TCR β or portion thereof carried or expressed by every lymphocyte of a population of an individual at a frequency of .001 percent or greater. In still another embodiment, "substantially equivalent" means that with ninety-nine percent probability a repertoire of nucleotide sequences will include a nucleotide sequence encoding an IgH or TCR β or portion thereof carried or expressed by every lymphocyte present at a frequency of .0001 percent or greater. In some embodiments, clonotype profiles are derived from samples comprising from 10⁵ to 10⁷ lymphocytes. Such numbers of lymphocytes may be obtained from peripheral blood samples of from 1-10 mL.
"Complementarity determining regions" (CDRs) mean regions of an immunoglobulin (i.e., antibody) or T cell receptor where the molecule complements an antigen's conformation, thereby determining the molecule's specificity and contact with a specific antigen. T cell receptors and immunoglobulins each have three CDRs: CDR1 and CDR2 are found in the variable (V) domain, and CDR3 includes some of V, all of diverse (D) (heavy chains only) and joint (J), and some of the constant (C) domains.
"Clonotype Database" means a collection of clonotypes formatted and arranged for ease and speed of searching, comparing and retrieving. In some embodiments, a clonotype database comprises a collection of clonotypes encoding the same region or segment of an immune receptor. In some embodiments, a clonotype database comprises clonotypes of clonotype profiles from a plurality of individuals. In some embodiments, a clonotype database comprises clonotypes of clonotype profiles of at least 10⁴ clonotypes from at least 10 individuals. In some embodiments, a clonotype database comprises at least 10⁶ clonotypes, or at least 10⁸ clonotypes, or at least 10⁹ clonotypes, or at least 10¹⁰ clonotypes. A clonotype database may be a public database containing clonotypes, such as IMGT database (www.imgt.org), e.g. described in Nucleic Acids Research, 31: 307-310 (2003). Clonotype databases may be in a FASTA format, and clonotype database entries may be searched or compared using a BLAST algorithm, e.g. Altschul et al, J. Mol. Biol., 215(3): 403-410 (1990), or like algorithm.
"Coalescing" means treating two clonotypes with sequence differences as the same by determining that such differences are due to experimental or measurement error and not due to genuine biological differences. In one aspect, a sequence of a higher frequency clonotype is compared to that of a lower frequency clonotype and if predetermined criteria are satisfied then the number of lower frequency clonotypes is added to that of the higher frequency clonotype and the lower frequency clonotype is thereafter disregarded. That is, the sequence read counts associated with the lower frequency clonotype are added to those of the higher frequency clonotype (and in some embodiments, the sequence associated with the lower frequency clonotype is removed from further consideration in the generation of a clonotype profile).
"Lymphoid or myeloid proliferative disorder" means any abnormal proliferative disorder in which one or more nucleotide sequences encoding one or more rearranged immune receptors can be used as a marker for monitoring such disorder. "Lymphoid or myeloid neoplasm" means an abnormal proliferation of lymphocytes or myeloid cells that may be malignant or non-malignant. A lymphoid cancer is a malignant lymphoid neoplasm. A myeloid cancer is a malignant myeloid neoplasm. Lymphoid and myeloid neoplasms are the result of, or are associated with, lymphoproliferative or myeloproliferative disorders, and include, but are not limited to, follicular lymphoma, chronic lymphocytic leukemia (CLL), acute lymphocytic leukemia (ALL), chronic myelogenous leukemia (CML), acute myelogenous leukemia (AML), Hodgkins's and non-Hodgkin's lymphomas, multiple myeloma (MM), monoclonal gammopathy of undetermined significance (MGUS), mantle cell lymphoma (MCL), diffuse large B cell lymphoma (DLBCL), myelodysplastic syndromes (MDS), T cell lymphoma, or the like, e.g. Jaffe et al, Blood, 112: 4384-4399 (2008); Swerdlow et al, WHO Classification of Tumours of Haematopoietic and Lymphoid Tissues (e. 4th) (IARC Press, 2008).
"Pecent homologous," "percent identical," or like terms used in reference to the comparison of a reference sequence and another sequence ("comparison sequence") mean that in an optimal alignment between the two sequences, the comparison sequence is identical to the reference sequence in a number of subunit positions equivalent to the indicated percentage, the subunits being nucleotides for polynucleotide comparisons or amino acids for polypeptide comparisons. As used herein, an "optimal alignment" of sequences being compared is one that maximizes matches between subunits and minimizes the number of gaps employed in constructing an alignment. Percent identities may be determined with commercially available implementations of algorithms, such as that described by Needleman and Wunsch, J. Mol. Biol., 48: 443-453 (1970)("GAP" program of Wisconsin Sequence Analysis Package, Genetics Computer Group, Madison, WI), or the like. Other software packages in the art for constructing alignments and calculating percentage identity or other measures of similarity include the "BestFit" program, based on the algorithm of Smith and Waterman, Advances in Applied Mathematics, 2: 482-489 (1981) (Wisconsin Sequence Analysis Package, Genetics Computer Group, Madison, WI). In other words, for example, to obtain a polynucleotide having a nucleotide sequence at least 95 percent identical to a reference nucleotide sequence, up to five percent of the nucleotides in the reference sequence may be deleted or substituted with another nucleotide, or a number of nucleotides up to five percent of the total number of nucleotides in the reference sequence may be inserted into the reference sequence.
"Polymerase chain reaction," or "PCR," means a reaction for the in vitro amplification of specific DNA sequences by the simultaneous primer extension of complementary strands of DNA. In other words, PCR is a reaction for making multiple copies or replicates of a target nucleic acid flanked by primer binding sites, such reaction comprising one or more repetitions of the following steps: (i) denaturing the target nucleic acid, (ii) annealing primers to the primer binding sites, and (iii) extending the primers by a nucleic acid polymerase in the presence of nucleoside triphosphates. Usually, the reaction is cycled through different temperatures optimized for each step in a thermal cycler instrument. Particular temperatures, durations at each step, and rates of change between steps depend on many factors well-known to those of ordinary skill in the art, e.g. exemplified by the references: McPherson et al, editors, PCR: A Practical Approach and PCR2: A Practical Approach (IRL Press, Oxford, 1991 and 1995, respectively). For example, in a conventional PCR using Taq DNA polymerase, a double stranded target nucleic acid may be denatured at a temperature >90°C, primers annealed at a temperature in the range 50-75°C, and primers extended at a temperature in the range 72-78°C. The term "PCR" encompasses derivative forms of the reaction, including but not limited to, RT-PCR, real-time PCR, nested PCR, quantitative PCR, multiplexed PCR, and the like. Reaction volumes range from a few hundred nanoliters, e.g. 200 nL, to a few hundred µL, e.g. 200 µL. "Reverse transcription PCR," or "RT-PCR," means a PCR that is preceded by a reverse transcription reaction that converts a target RNA to a complementary single stranded DNA, which is then amplified, e.g. Tecott et al, U.S. patent 5,168,038. "Real-time PCR" means a PCR for which the amount of reaction product, i.e. amplicon, is monitored as the reaction proceeds. There are many forms of real-time PCR that differ mainly in the detection chemistries used for monitoring the reaction product, e.g. Gelfand et al, U.S. patent 5,210,015 ("taqman"); Wittwer et al, U.S. patents 6,174,670 and 6,569,627 (intercalating dyes); Tyagi et al, U.S. patent 5,925,517 (molecular beacons). Detection chemistries for real-time PCR are reviewed in Mackay et al, Nucleic Acids Research, 30: 1292-1305 (2002). "Nested PCR" means a two-stage PCR wherein the amplicon of a first PCR becomes the sample for a second PCR using a new set of primers, at least one of which binds to an interior location of the first amplicon. As used herein, "initial primers" in reference to a nested amplification reaction mean the primers used to generate a first amplicon, and "secondary primers" mean the one or more primers used to generate a second, or nested, amplicon. "Multiplexed PCR" means a PCR wherein multiple target sequences (or a single target sequence and one or more reference sequences) are simultaneously carried out in the same reaction mixture, e.g. Bernard et al, Anal. Biochem., 273: 221-228 (1999)(two-color real-time PCR). Usually, distinct sets of primers are employed for each sequence being amplified. Typically, the number of target sequences in a multiplex PCR is in the range of from 2 to 50, or from 2 to 40, or from 2 to 30. "Quantitative PCR" means a PCR designed to measure the abundance of one or more specific target sequences in a sample or specimen. Quantitative PCR includes both absolute quantitation and relative quantitation of such target sequences. Quantitative measurements are made using one or more reference sequences or internal standards that may be assayed separately or together with a target sequence. The reference sequence may be endogenous or exogenous to a sample or specimen, and in the latter case, may comprise one or more competitor templates. Typical endogenous reference sequences include segments of transcripts of the following genes: β-actin, GAPDH, β₂-microglobulin, ribosomal RNA, and the like. Techniques for quantitative PCR are well-known to those of ordinary skill in the art, as exemplified in the following references : Freeman et al, Biotechniques, 26: 112-126 (1999); Becker-Andre et al, Nucleic Acids Research, 17: 9437-9447 (1989); Zimmerman et al, Biotechniques, 21: 268-279 (1996); Diviacco et al, Gene, 122: 3013-3020 (1992); Becker-Andre et al, Nucleic Acids Research, 17: 9437-9446 (1989); and the like.
"Primer" means an oligonucleotide, either natural or synthetic that is capable, upon forming a duplex with a polynucleotide template, of acting as a point of initiation of nucleic acid synthesis and being extended from its 3' end along the template so that an extended duplex is formed. Extension of a primer is usually carried out with a nucleic acid polymerase, such as a DNA or RNA polymerase. The sequence of nucleotides added in the extension process is determined by the sequence of the template polynucleotide. Usually primers are extended by a DNA polymerase. Primers usually have a length in the range of from 14 to 40 nucleotides, or in the range of from 18 to 36 nucleotides. Primers are employed in a variety of nucleic amplification reactions, for example, linear amplification reactions using a single primer, or polymerase chain reactions, employing two or more primers. Guidance for selecting the lengths and sequences of primers for particular applications is well known to those of ordinary skill in the art, as evidenced by the following references : Dieffenbach, editor, PCR Primer: A Laboratory Manual, 2nd Edition (Cold Spring Harbor Press, New York, 2003).
"Quality score" means a measure of the probability that a base assignment at a particular sequence location is correct. A variety methods are well known to those of ordinary skill for calculating quality scores for particular circumstances, such as, for bases called as a result of different sequencing chemistries, detection systems, base-calling algorithms, and so on. Generally, quality score values are monotonically related to probabilities of correct base calling. For example, a quality score, or Q, of 10 may mean that there is a 90 percent chance that a base is called correctly, a Q of 20 may mean that there is a 99 percent chance that a base is called correctly, and so on. For some sequencing platforms, particularly those using sequencing-by-synthesis chemistries, average quality scores decrease as a function of sequence read length, so that quality scores at the beginning of a sequence read are higher than those at the end of a sequence read, such declines being due to phenomena such as incomplete extensions, carry forward extensions, loss of template, loss of polymerase, capping failures, deprotection failures, and the like.
"Sequence read" means a sequence of nucleotides determined from a sequence or stream of data generated by a sequencing technique, which determination is made, for example, by means of base-calling software associated with the technique, e.g. base-calling software from a commercial provider of a DNA sequencing platform. A sequence read usually includes quality scores for each nucleotide in the sequence. Typically, sequence reads are made by extending a primer along a template nucleic acid, e.g. with a DNA polymerase or a DNA ligase. Data is generated by recording signals, such as optical, chemical (e.g. pH change), or electrical signals, associated with such extension. Such initial data is converted into a sequence read.

## Claims

1. A method of selecting one or more patient-specific clonotypes correlated with a lymphoid neoplasm for monitoring a minimal residual disease thereof, the method comprising the steps of:
(a) amplifying molecules of nucleic acid from T-cells and/or B-cells of a sample obtained from a patient, the molecules of nucleic acid comprising recombined DNA sequences from T-cell receptor genes or immunoglobulin genes;
(b) sequencing the amplified molecules of nucleic acid to form a clonotype profile;
(c) comparing clonotypes from the clonotype profile to clonotypes of a clonotype database, wherein the clonotype database comprises clonotypes of clonotype profiles of at least 10⁴ clonotypes from at least 10 individuals other than the patient, to determine a presence, absence and/or level in the clonotype database of each clonotype from the clonotype profile, wherein the step of comparing includes counting a clonotype as present in the clonotype database if a clonotype in the clonotype database is a member of the same clan as the clonotype of the clonotype profile, and a clonotype is considered to be a member of the same clan if
(i) the clonotype of the clonotype database is at least ninety percent identical to the clonotype of the clonotype profile;
(ii) the clonotype of the clonotype database and the clonotype of the clonotype profile each comprise recombined sequences from immunoglobulin heavy chain and are related by a VH replacement;
(iii) the clonotype of the clonotype database and the clonotype of the clonotype profile have identically mutated V region and J region but have a different NDN region;
(iv) the clonotype of the clonotype database and the clonotype of the clonotype profile each comprise recombined sequences from immunoglobulin heavy chain and are related by hypermutation; or
(v) if the clonotypes have undergone one or more insertions and/or deletions of from 1-10 bases; and
(d) selecting the one or more patient-specific clonotypes for monitoring the minimal residual disease which are each correlated with the lymphoid neoplasm and which are each:
i) absent from the clonotype database; or
ii) at a level in the clonotype database below a predetermined frequency.

2. The method of claim 1, wherein the one or more patient-specific clonotypes are each 25 to 400 nucleotide sequences encoding a segment of an immune receptor or immune receptor component selected from the group consisting of a VDJ rearrangement of IgH, a DJ rearrangement of IgH, a VJ rearrangement of IgK, a VJ rearrangement of IgL, a VDJ rearrangement of TCR β, a DJ rearrangement of TCR β, a VJ rearrangement of TCR α, a VJ rearrangement of TCR γ, a VDJ rearrangement of TCR δ, and a VD rearrangement of TCR δ.

3. The method of claim 1, wherein the step of comparing includes aligning sequences of the clonotypes of the clonotype profile with sequences of the clonotypes of the clonotype database.

## Patentansprüche

1. Verfahren zum Selektieren eines oder mehrerer patientenspezifischer Klonotypen, die mit einem lymphoiden Neoplasma korreliert sind, zum Überwachen einer minimalen Resterkrankung davon, wobei das Verfahren die Schritte umfasst:
(a) Amplifizieren von Nukleinsäuremolekülen aus T-Zellen und/oder B-Zellen einer von einem Patienten erhaltenen Probe, wobei die Nukleinsäuremoleküle rekombinierte DNA-Sequenzen von T-Zell-Rezeptorgenen oder Immunglobulin-Genen umfassen;
(b) Sequenzieren der amplifizierten Nukleinsäuremoleküle, um ein Klonotypprofil zu bilden;
(c) Vergleichen von Klonotypen des Klonotypprofils mit Klonotypen einer Klonotypdatenbank, wobei die Klonotypdatenbank Klonotypen von Klonotypprofilen von mindestens 10⁴ Klonotypen von mindestens 10 Individuen, die sich vom Patienten unterscheiden, umfasst, um eine Anwesenheit, eine Abwesenheit und/oder einen Spiegel in der Klonotypdatenbank jedes Klonotyps vom Klonotypprofil zu bestimmen, wobei der Schritt des Vergleichens ein Zählen eines Klonotyps als in der Klonotypdatenbank anwesend umfasst, wenn ein Klonotyp in der Klonotypdatenbank ein Mitglied desselben Stamms wie der Klonotyp des Klonotypprofils ist, und wobei ein Klonotyp als ein Mitglied desselben Stamms angesehen wird, wenn
(i) der Klonotyp der Klonotypdatenbank zu mindestens neunzig Prozent mit dem Klonotyp des Klonotypprofils identisch ist;
(ii) der Klonotyp der Klonotypdatenbank und der Klonotyp des Klonotypprofils jeweils rekombinierte Sequenzen von einer schweren Kette von Immunglobulin umfassen und durch einen VH-Ersatz in Beziehung stehen;
(iii) der Klonotyp der Klonotypdatenbank und der Klonotyp des Klonotypprofils eine identische mutierte V-Region und J-Region aufweisen, aber eine andere NDN-Region aufweisen;
(iv) der Klonotyp der Klonotypdatenbank und der Klonotyp des Klonotypprofils jeweils rekombinierte Sequenzen von einer schweren Kette von Immunglobulin umfassen und durch Hypermutation in Beziehung stehen; oder
(v) wenn die Klonotypen einer oder mehreren Insertionen und/oder Deletionen von 1-10 Basen unterzogen wurden; und
(d) Auswählen des einen oder der mehreren patientenspezifischen Klonotypen zum Überwachen der minimalen Resterkrankung, die jeweils mit dem lymphoiden Neoplasma korreliert sind, und die jeweils:
i) in der Klonotypdatenbank abwesend sind; oder
ii) in der Klonotypdatenbank auf einem Spiegel unterhalb einer vorbestimmten Häufigkeit sind.

2. Verfahren nach Anspruch 1, wobei der eine oder die mehreren patientenspezifischen Klonotypen jeweils Sequenzen mit 25 bis 400 Nukleotiden sind, die für ein Segment eines Immunrezeptors oder einer Immunrezeptorkomponente kodieren, ausgewählt aus der Gruppe bestehend aus einer VDJ Umordnung von IgH, einer DJ Umordnung von IgH, einer VJ Umordnung von IgK , einer VJ Umordnung von IgL, einer VDJ Umordnung von TCR β, einer DJ Umordnung von TCR β, einer VJ Umordnung von TCR α, einer VJ Umordnung von TCR γ, einer VDJ Umordnung von TCR δ und einer VD Umordnung von TCR δ.

3. Verfahren nach Anspruch 1, wobei der Schritt des Vergleichens ein Ausrichten von Sequenzen der Klonotypen des Klonotypprofils mit Sequenzen der Klonotypen der Klonotypdatenbank umfasst.

## Revendications

1. Procédé de sélection d'un ou plusieurs clonotypes spécifiques à un patient en corrélation avec un néoplasme lymphoïde pour surveiller une maladie résiduelle minimale de celui-ci, le procédé comprenant les étapes consistant à :
(a) amplifier des molécules d'acide nucléique provenant de lymphocytes T et/ou de lymphocytes B d'un échantillon obtenu auprès d'un patient, les molécules d'acide nucléique comprenant des séquences d'ADN recombinées provenant de gènes de récepteurs de lymphocytes T ou de gènes d'immunoglobuline ;
(b) séquencer les molécules amplifiées d'acide nucléique pour former un profil clonotypique ;
(c) comparer des clonotypes du profil clonotypique à des clonotypes d'une base de données de clonotypes, dans lequel la base de données de clonotypes comprend des clonotypes de profils clonotypiques d'au moins 10⁴ clonotypes d'au moins 10 individus autres que le patient, afin de déterminer une présence, une absence et/ou un niveau dans la base de données de clonotypes de chaque clonotype du profil clonotypique, dans lequel l'étape de comparaison comprend le comptage d'un clonotype présent dans la base de données de clonotypes si un clonotype de la base de données de clonotypes est un membre de la même famille que le clonotype du profil clonotypique, et un clonotype est considéré comme étant un membre de la même famille si
(i) le clonotype de la base de données de clonotypes est au moins quatre-vingt-dix pour cent identique au clonotype du profil clonotypique ;
(ii) le clonotype de la base de données de clonotypes et le clonotype du profil clonotypique comprennent chacun des séquences recombinées à partir de la chaîne lourde d'immunoglobuline et sont liés par un remplacement de VH ;
(iii) le clonotype de la base de données de clonotypes et le clonotype du profil clonotypique ont une région V et une région J mutées de manière identique, mais ont une région NDN différente ;
(iv) le clonotype de la base de données de clonotypes et le clonotype du profil clonotypique comprennent chacun des séquences recombinées de la chaîne lourde d'immunoglobuline et sont liés par hypermutation ; ou
(v) si les clonotypes ont subi une ou plusieurs insertions et/ou délétions de 1 à 10 bases ; et
(d) sélectionner les un ou plusieurs clonotypes spécifiques à un patient pour surveiller la maladie résiduelle minimale qui sont chacun mis en corrélation avec le néoplasme lymphoïde et qui sont chacun :
i) absent de la base de données de clonotypes ; ou
ii) à un niveau dans la base de données de clonotypes inférieur à une fréquence prédéterminée.

2. Procédé selon la revendication 1, dans lequel les un ou plusieurs clonotypes spécifiques à un patient sont chacun 25 à 400 séquences nucléotidiques codant pour un segment d'un récepteur immunitaire ou d'un composant de récepteur immunitaire choisi dans le groupe constitué d'un réarrangement VDJ d'IgH, d'un réarrangement DJ d'IgH, d'un réarrangement VJ d'IgK, d'un réarrangement VJ d'IgL, d'un réarrangement VDJ de TCR β, d'un réarrangement DJ de TCR β, d'un réarrangement VJ de TCR α, d'un réarrangement VJ de TCR γ, d'un réarrangement VDJ de TCR δ, et d'un réarrangement VD de TCR δ.

3. Procédé selon la revendication 1, dans lequel l'étape de comparaison comprend l'alignement des séquences des clonotypes du profil clonotypique avec des séquences des clonotypes de la base de données de clonotypes.
